# EUROPEAN PATENT APPLICATION

(11) **EP 3 439 060 A1**
(43) Date of publication of application: **06.02.2019**
(21) Application number: 17774329.1
(22) Date of filing: 15.03.2017
(51) Int. Cl.: H01L 51/50, C07D 209/88, C07D 401/14, C07D 403/14, C07D 405/14, C07D 409/14

(54) **ORGANIC ELECTROLUMINESCENT ELEMENT**

(30) Priority: 28.03.2016 JP 2016064233
(71) Applicant: Nippon Steel & Sumikin Chemical Co., Ltd., Chiyoda-ku Tokyo 101-0021 (JP)
(72) Inventor: UEDA Tokiko, Tokyo 101-0021 (JP); OGAWA Junya, Tokyo 101-0021 (JP); TADA Masashi, Tokyo 101-0021 (JP)
(74) Representative: Vossius & Partner Patentanwälte Rechtsanwälte mbB
(86) International application number: PCT/JP2017/010409
(87) International publication number: WO 2017/169785

(57) **Abstract**

Provided is an organic EL element which is low voltage and has practical use utility while exhibiting high light emission efficiency and drive stability. Thus, an organic electroluminescent element obtained by layering a positive electrode, an organic layer and a negative electrode on a substrate, wherein at least one layer among the organic layers contains (i) a carbazole compound represented by general formula (1), and (ii) a carborane compound having one or more divalent carborane compounds and an aromatic group substituted for a carborane compound. Herein, L¹ is an aromatic hydrocarbon group, an aromatic heterocyclic group, or a linked aromatic group configured by linking 2-6 of said aromatic rings, p is an integer of 1-3, and m is an integer of 2-4.

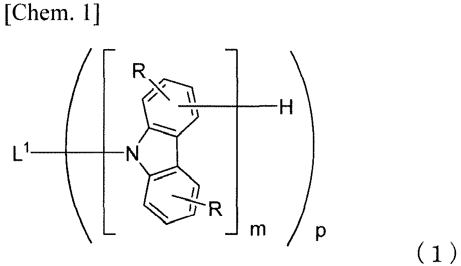

## Description

### FIELD

The present invention relates to an organic electroluminescent device (hereinafter, referred to as "organic EL device") and in particular relates to an organic EL device having an organic layer comprising a plurality of compounds.

### BACKGROUND

When a voltage is applied to an organic EL device, a hole is injected from an anode into a light-emitting layer, and an electron is injected from a cathode into the layer. Then, in the light-emitting layer, the hole and the electron thus injected recombine to produce an exciton. At this time, according to the statistical law of electron spins, singlet excitons and triplet excitons are produced at a ratio of 1:3. The internal quantum efficiency of a fluorescent emission-type organic EL device using light emission by a singlet exciton is said to be at most 25%. Meanwhile, it has been known that the internal quantum efficiency of a phosphorescent emission-type organic EL device using light emission by a triplet exciton can be improved to 100% when intersystem crossing from a singlet exciton is efficiently performed.

Recently, highly efficient organic EL devices utilizing delayed fluorescence have been developed. For example, Patent Literature 1 discloses an organic EL device utilizing a TTF (Triplet-Triplet Fusion) mechanism, which is one type of mechanism of delayed fluorescence. Patent Literature 2 discloses an organic EL device utilizing TADF (Thermally Activated Delayed Fluorescence). Though both are means capable of enhancing internal quantum efficiency, a further improvement in lifetime characteristics has been demanded in the same manner as for phosphorescent emission-type devices.

### [CITATION LIST]

### [PATENT LITERATURE]

Patent Literature 1: WO2010/134350 A1
Patent Literature 2: WO2011/070963 A1
Patent Literature 3: JP 2005-162709 A
Patent Literature 4: JP 2005-166574 A
Patent Literature 5: US2012/0319088 A1
Patent Literature 6: WO2013/094834 A1
Patent Literature 7: US2009/0167162 A1
Patent Literature 8: WO2015/137202 A1

Patent Literature 3 to 8 disclose the use of a carborane compound as a host material. Patent Literature 8 discloses the use of a specific carborane compound as a delayed fluorescent material, the use of biscarbazole compounds as delayed fluorescent materials, and the use of a carborane compound as a host material in a light-emitting layer, but does not teach the use of a carborane compound mixed with a carbazole compound in an organic layer other than a light-emitting layer or as a host material in a light-emitting layer.

### SUMMARRY

### [TECHNICAL PROBLEM]

In order to apply an organic EL device to a display device, such as a flat panel display, or a light source, the luminous efficiency of the device needs to be improved, and at the same time, stability at the time of driving needs to be sufficiently secured. In view of the above-mentioned present circumstances, an object of the present invention is to provide a practically useful organic EL device having high efficiency and high driving stability while having a low driving voltage.

### [SOLUTION TO PROBLEM]

The present invention relates to an organic electroluminescent device comprising a substrate having stacked thereon an anode, an organic layer and a cathode, wherein at least one layer of the organic layer comprises (i) a compound represented by the following general formula (1) and (ii) a compound represented by the following general formula (2):

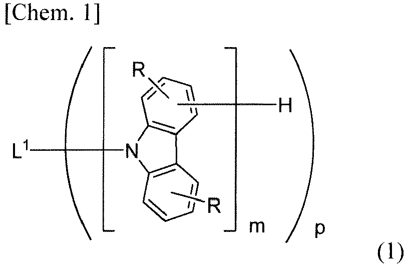

In general formula (1), L¹ is a *p*-valent group, and is a substituted or unsubstituted aromatic hydrocarbon group having 6 to 30 carbon atoms, a substituted or unsubstituted heteroaromatic ring group having 3 to 30 carbon atoms, or a substituted or unsubstituted linked aromatic group formed by linking 2 to 6 of the aromatic rings thereof (which refer to aromatic rings of the substituted or unsubstituted aromatic hydrocarbon group or substituted or the unsubstituted aromatic heterocyclic group).

Each R independently represents hydrogen, a substituted or unsubstituted aromatic hydrocarbon group having 6 to 30 carbon atoms, a substituted or unsubstituted heteroaromatic ring group having 3 to 30 carbon atoms, a substituted or unsubstituted linked aromatic group formed by linking 2 to 6 of the rings thereof, an alkyl group having 1 to 12 carbon atoms, a diarylamino group having 12 to 44 carbon atoms, a cyano group, a nitro group, or a fluoro group. The alkyl group may be linear, branched, or cyclic.
*p* is a substitution number, and represents an integer of 1 to 3. *m* is a repeating number, and independently represents an integer of 2 to 4.

When L¹ or R is a heteroaromatic ring group, the heteroaromatic ring group is not a carbazolyl group or a carbazole ring-containing group.

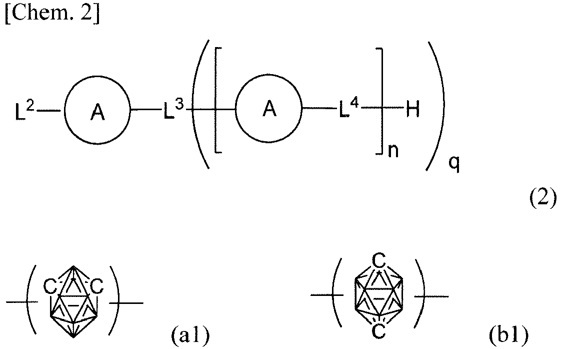

In general formula (2), ring A be a divalent carborane group of C₂B₁₀H₁₀ represented by formula (a1) or formula (b1). However, when a plurality of rings A are present in a molecule, the plurality of rings A may be the same or different from each other. *q* is a substitution number, and represents an integer of 1 to 4. *n* is a repeating number, and independently represents an integer of 0 to 2.

L² represents a substituted or unsubstituted aromatic hydrocarbon group having 6 to 30 carbon atoms, a substituted or unsubstituted heteroaromatic ring group having 3 to 30 carbon atoms, or a substituted or unsubstituted linked monovalent aromatic group formed by linking 2 to 6 aromatic rings thereof.

L³ is a single bond or a (*q* + 1)-valent group, and represents a substituted or unsubstituted aromatic hydrocarbon group having 6 to 30 carbon atoms, a substituted or unsubstituted heteroaromatic ring group having 3 to 30 carbon atoms, or a substituted or unsubstituted linked aromatic group formed by linking 2 to 6 aromatic rings thereof. However, when q = 1 and n = 1, L³ represents a single bond, an aromatic heterocyclic group, or a linked aromatic group comprising at least one aromatic heterocyclic group.

L⁴ is independently a single bond or divalent group. The divalent group represents a substituted or unsubstituted aromatic hydrocarbon group having 6 to 30 carbon atoms, a substituted or unsubstituted heteroaromatic ring group having 3 to 30 carbon atoms, or a substituted or unsubstituted linked aromatic group formed by linking 2 to 6 of the substituted or unsubstituted aromatic rings.

In general formula (1), *p* is an integer of 1 or 2 and *m* is independently an integer of 2 or 3. It is preferable that all binding structures between carbazolyl groups are binding structures represented by formula (d1) or binding structures represented by formula (c1) and formula (d1). The latter binding structures are more preferable.

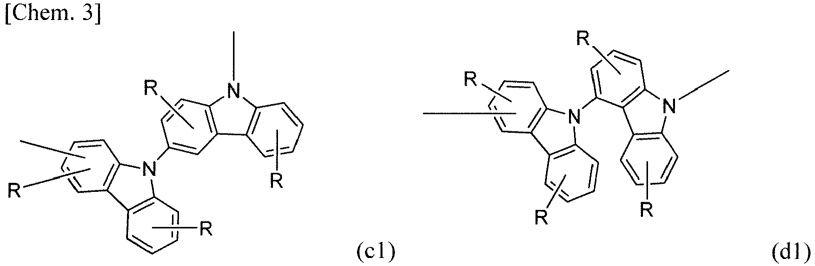

In general formula (1), L¹ is preferably a *p*-valent group formed by removing p hydrogen atoms from any one of formulae (3) to (6), and is more preferably a *p*-valent group formed by removing p hydrogen atoms from any one of formulae (3), (4), and (5).

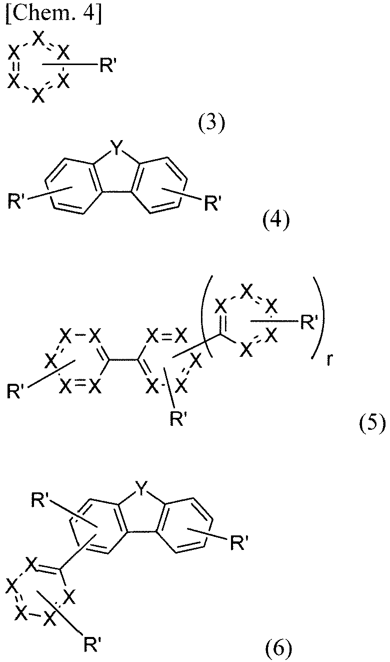

In formulae (3) to (6), each X independently represents CH or nitrogen, each R' independently represents hydrogen, a substituted or unsubstituted aromatic hydrocarbon group having 6 to 30 carbon atoms, a substituted or unsubstituted heteroaromatic ring group having 3 to 30 carbon atoms, an alkyl group having 1 to 12 carbon atoms, a diarylamino group having 12 to 44 carbon atoms, a cyano group, a nitro group, or a fluoro group. In formulae (4) and (6), Y represents oxygen or sulfur, and in formulae (5), *r* represents an integer of 0 to 2.

In general formula (1), the total of *m* can be an integer of 2 to 6.

In general formula (2), it is preferable that ring A be a divalent carborane group of C₂B₁₀H₁₀ represented by formula (a1), the aromatic rings of L² and L³, which bond to ring A, be the same, or L² and L³ be a substituted or unsubstituted dibenzofuranyl group or a substituted or unsubstituted dibenzothiophenyl group.

The organic layer comprising a compound represented by the following general formula (1) and a compound represented by the following general formula (2) is preferably at least one layer selected from a light-emitting layer containing a luminescent dopant, an electron-blocking layer, and a hole-blocking layer. It is more preferable that the organic layer comprising two or more compounds be a light-emitting layer containing a luminescent dopant and contain the two compounds as host materials.

Further, the luminescent dopant is preferably a delayed fluorescent dopant or an organometallic complex comprising at least one metal selected from ruthenium, rhodium, palladium, silver, rhenium, osmium, iridium, platinum, and gold.

In order to improve the characteristics of the devices, it is important to prevent the leakage of excitons and charge into adjacent layers. Improving deviation of light emitting areas in a light-emitting layer is effective to prevent such leakage of charge/excitons. For this purpose, it is necessary to control the injection/transportation amount of both types of charge (electron/hole) in a material constituting an organic layer to within a preferable range.

Regarding a carbazole compound represented by general formula (1), the stability of the skeleton thereof is high, and the electron/hole injection/transportation property thereof can be controlled using an isomer or a substituent to some extent. However, it is difficult to control the injection/transportation amount of both types of the compound alone to a preferable range. Regarding a carborane compound represented by general formula (2), the lowest unoccupied molecular orbital (LUMO), which influences the electron injection/transportation property, is widely distributed throughout the molecule thereof, and thus, the electron injection/transportation property of a device is highly controllable. Additionally, since the skeleton stability is high in the same manner as the carbazole compound, the charge injection amount into an organic layer can be precisely controlled by the use of the carborane compound mixed with a biscarbazole compound. In particular, by the use thereof in a light-emitting layer or a charge blocking layer, the balance of both types of charges can be controlled. In the cases of delayed fluorescent EL devices and phosphorescent EL devices, since each of the compounds has excitation energy (singlet and triplet) high enough to confine excitation energy generated in a light-emitting layer, there is no energy outflow from inside the light-emitting layer, and high efficiency and long life can be achieved at low voltages.

### BRIEF DESCTIPTIN OF DRAWINGS

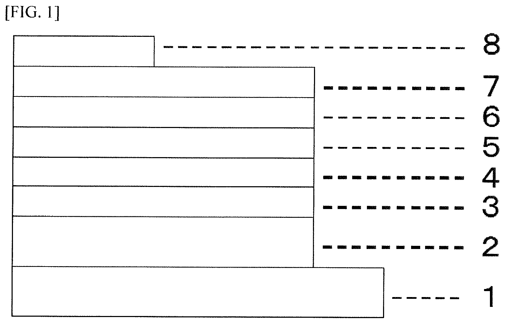

[FIG. 1] FIG. 1 schematically illustrates the cross-section of an example of an organic EL device.

### DESCRIPTION OF EMBODIMENTS

The organic electroluminescent device of the present invention, comprising a substrate having stacked thereon an anode, an organic layer, and a cathode, comprises (i) a compound represented by general formula (1) and (ii) a compound represented by general formula (2) in at least one layer of the organic layer. The compounds of general formula (1) and general formula (2) each may be one compound, or both or either one may be two or more compounds. These compounds are present as a mixture in the organic layer. The ratio of the compound represented by general formula (1) is desirably 30 wt% or more with respect to the total of the compound represented by general formula (1) and the compound represented by general formula (2). This ratio is more preferably 35 to 95 wt%, and further preferably 40 to 90 wt%.

In general formula (1), L¹ is a *p*-valent aromatic group. The aromatic group refers to an aromatic hydrocarbon group, an aromatic heterocyclic group, or a linked aromatic group formed by linking 2 to 6 of the aromatic rings thereof. The aromatic ring refers to aromatic hydrocarbon rings, heteroaromatic rings, or both.

The *p*-valent aromatic hydrocarbon group is a substituted or unsubstituted aromatic hydrocarbon group having 6 to 30 carbon atoms. The aromatic heterocyclic group is a substituted or unsubstituted aromatic heterocyclic group having 3 to 30 carbon atoms. The linked aromatic group is a linked aromatic group formed by linking 2 to 6 aromatic rings of the aromatic hydrocarbon group and the aromatic heterocyclic group via direct bonding, and is a substituted or unsubstituted linked aromatic group. The *p*-valent aromatic hydrocarbon group is preferably a substituted or unsubstituted aromatic hydrocarbon group having 6 to 18 carbon atoms, an aromatic heterocyclic group other than a substituted or unsubstituted carbazolyl group having 3 to 17 carbon atoms, or a substituted or unsubstituted linked aromatic group formed by linking 2 to 4 of the rings thereof.

In general formula (1), each R independently represents hydrogen, a substituted or unsubstituted aromatic hydrocarbon group having 6 to 30 carbon atoms, a substituted or unsubstituted aromatic heterocyclic group having 3 to 30 carbon atoms, a substituted or unsubstituted linked aromatic group formed by linking 2 to 6 of the rings thereof, an alkyl group having 1 to 12 carbon atoms, a diarylamino group having 12 to 44 carbon atoms, a cyano group, a nitro group, or a fluoro group. Each R preferably is a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 4 of the rings thereof. The alkyl group may be linear, branched, or cyclic.

When L¹ or R is an aromatic heterocyclic group, the aromatic heterocyclic group does not comprise a carbazolyl group. The carbazolyl group is understood to encompass typical carbazolyl groups, divalent or higher valent carbazolyl groups, and carbazole ring-containing groups which may have a substituent.

When L¹ and R in general formula (1), (c1), and (d1) are an unsubstituted aromatic hydrocarbon group, an aromatic heterocyclic group other than an unsubstituted carbazolyl group, or an unsubstituted linked aromatic group, specific examples thereof include *p*-valent or monovalent groups formed by removing hydrogen from benzene, pentalene, indene, naphthalene, azulene, heptalene, octalene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, helicene, hexaphene, rubicene, coronene, trinaphthylene, heptaphene, pyranthrene, and other aromatic hydrocarbon compounds, furan, benzofuran, isobenzofuran, xanthene, oxanthrene, dibenzofuran, peri-xanthenoxanthene, thiophene, thioxanthene, thianthrene, phenoxathiin, thionaphthene, isothianaphthene, thiophthene, thiophanthrene, dibenzothiophene, pyrrole, pyrazole, tellurazole, selenazole, thiazole, isothiazole, oxazole, furazan, pyridine, pyrazine, pyrimidine, pyridazine, triazine, indolizine, indole, isoindole, indazole, purine, quinolizine, isoquinoline, imidazole, naphthyridine, phthalazine, quinazoline, benzodiazepine, quinoxaline, cinnoline, quinoline, pteridine, phenanthridine, acridine, perimidine, phenanthroline, phenazine, carboline, , phenotellurazine, phenoselenazine, phenothiazine, phenoxazine, anthyridine, benzothiazole, benzimidazole, benzoxazole, benzisooxazole, benzisothiazole, and other heteroaromatic ring compounds, and aromatic compounds each composed of a plurality of linked aromatic groups of the above aromatic compounds

In the case of a linked aromatic group formed by linking a plurality of linked aromatic groups, the number of linked groups is 2 to 6, preferably 2 to 4. The linked aromatic groups may be the same or different.

Specific examples of the linked aromatic group include *p*-valent or monovalent groups formed by removing hydrogen from biphenyl, terphenyl, quaterphenyl, bipyridine, bipyrimidine, bitriazine, terpyridine, bistriazylbenzene, binaphthalene, phenylpyridine, diphenylpyridine, triphenylpyridine, phenylpyrimidine, diphenylpyrimidine, triphenylpyrimidine, phenyltriazine, diphenyltriazine, triphenyltriazine, phenylnaphthalene, diphenylnaphthalene, phenyldibenzofuran, phenyldibenzothiophene, dibenzofuranylpyridine, dibenzothiophenylpyridine, and other aromatic compounds.

When the aromatic hydrocarbon group, aromatic heterocyclic group, or linked aromatic group has a substituent, the substituent may be selected from an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group 1 to 20 having carbon atoms, a cyano group, a nitro group, a fluoro group, and a tosyl group. The substituent is preferably selected from an alkyl group having 1 to 12 carbon atoms, an aralkyl group having 7 to 20 carbon atoms, a diarylamino group having 12 to 30 carbon atoms, an alkoxy group having 1 to 10 carbon atoms, a cyano group, a fluoro group, and a tosyl group. The alkyl group amy be linear, branched, or cyclic.

Specific examples of the substituent include methyl, ethyl, propyl, butyl, pentyl, cyclopentyl, hexyl, cyclohexyl, heptyl, octyl, nonyl, decyl, undecyl, dodecyl, tridecyl, tetradecyl, pentadecyl, hexadecyl, heptadecyl, octadecyl, nonadecyl, icosyl, and other alkyl groups, phenylmethyl, phenylethyl, phenylicosyl, naphthylmethyl, anthranilmethyl, phenanthrenylmethyl, pyrenymethyl, and other aralkyl groups, vinyl, propenyl, butenyl, pentenyl, decenyl, icosenyl, and other alkenyl groups, ethynyl, propargyl, buthynyl, pentynyl, decynyl, icosynyl, and other alkynyl groups, dimethylamino, ethylmethylamino, diethylamino, dipropylamino, dibutylamino, dipentynylamino, didecylamino, diicosylamino, and other dialkylamino groups, diphenylamino, naphthylphenylamino, dinaphthylamino, dianthranylamino, diphenanthrenylamino, dipyrenylamino, and other diarylamino groups, diphenylmethylamino, diphenylethylamino, phenylmethylphenylethylamino, dinaphthylmethylamino, dianthranilmethylamino, diphenanthrenylmethylamino, and other diaralkylamino groups, acetyl, propionyl, butyryl, valeryl, benzoyl, and other acyl groups, acetyloxy, propionyloxy, butyryloxy, valeryloxy, benzoyloxy, and other acyloxy groups, methoxy, ethoxy, propoxy, butoxy, pentoxy, hexoxy, heptoxy, octoxy, nonyloxy, decanyloxy, and other alkoxy groups, methoxycarbonyl, ethoxycarbonyl , propoxycarbonyl, butoxycarbonyl, pentoxycarbonyl, and other akoxycarbonyl groups, methoxycarbonyloxy, ethoxycarbonyloxy, propoxycarbonyloxy, butoxycarbonyloxy, pentoxycarbonyloxy, and other akoxycarbonyloxy groups, methylsulfonyl, ethylsulfonyl, propylsulfonyl, butylsulfonyl, pentylsulfonyl, and other alkyl sulfoxy groups, cyano group, nitro group, fluoro group, and a tosyl group. The substituent is preferably selected from methyl, ethyl, propyl, butyl, pentyl, hexyl, heptyl, octyl, nonyl, decyl, and other alkyl groups having 1 to 12 carbon atoms, phenylmethyl, phenylethyl, naphthylmethyl, anthranilmethyl, phenanthrenylmethyl, pyrenymethyl, and other aralkyl groups having 7 to 20 carbon atoms, methoxy, ethoxy, propoxy, butoxy, pentoxy, hexoxy, heptoxy, octoxy, nonyloxy, decanyloxy, and other alkoxy groups having 1 to 10 carbon atoms, diphenylamino, naphthylphenylamino, dinaphthylamino, dianthranylamino, diphenanthrenylamino, and other diarylamino groups having two aromatic hydrocarbon groups having 6 to 15 carbon atoms, cyano group, fluoro group, and tosyl group.

As used herein, the term "linked aromatic group" refers to a group composed of a plurality of linked single rings or aromatic rings (aromatic hydrocarbon rings, heteroaromatic rings, or both) of aromatic compounds of condensed ring structures. The linked aromatic groups refer to linked aromatic rings of aromatic groups via direct bonds. When the aromatic groups are substituted aromatic groups, no substituents are aromatic groups.

The linked aromatic groups may be linear or branched. The aromatic rings to be linked may be the same or different, may have either or both of an aromatic hydrocarbon ring and a heteroaromatic ring, and may have a substituent.

Herein, the number of carbon atoms calculated is understood to exclude the number of carbon atoms of substituents. However, it is preferable that the total number of carbon atoms including the carbon atoms of the substituents be within the above ranges of the number of carbon atoms. The number of carbon atoms of the linked aromatic groups is understood to be the total number of carbon atoms of linked aromatic hydrocarbon groups and aromatic heterocyclic groups.

When the linked aromatic group is a monovalent group, the linking from may be, for example, as follows:

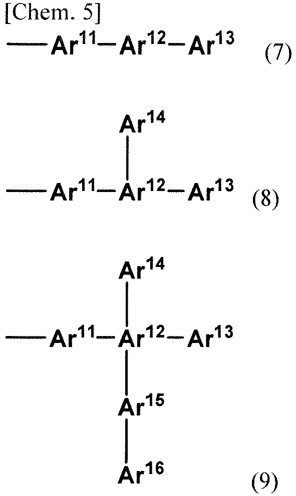

When the linked aromatic group is a divalent group, the linking from may be, for example, as follows. When the linked aromatic group is a trivalent or higher valent group, the linking from can be understood from the above.

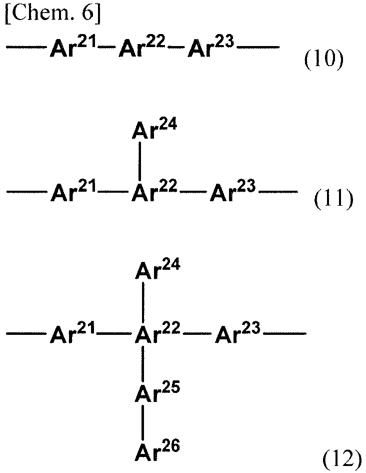

In formulae (7) to (12), Ar¹¹ to Ar¹⁶ and Ar²¹ to Ar²⁶ represent substituted or unsubstituted aromatic rings (aromatic groups), and ring-forming atoms of the aromatic groups bond together via direct bonding. The bonds start from the ring-forming atoms of the aromatic groups. The aromatic rings (aromatic groups) refer to aromatic hydrocarbon groups or aromatic heterocyclic groups, and may be monovalent or higher valent groups.

In formulae (7) to (12), the bond starts from Ar¹¹, Ar²¹, or Ar²³, but can start from another aromatic ring. In the case of divalent or higher valent group, two or more bonds start from one aromatic group.

When R in general formula (1), (c1), and (d1) is an alkyl group having 1 to 12 carbon atoms or an diarylamino group having 12 to 44 carbon atoms, specific examples thereof include methyl, ethyl, propyl, butyl, *tert*-butyl, pentyl, isopentyl, cyclopentyl, hexyl, cyclohexyl, heptyl, octyl, nonyl, decyl, undecyl, dodecyl, cyclohexyl, and other alkyl groups, diphenylamino, naphthylphenylamino, dinaphthylamino, dianthranylamino, diphenanthrenylamin, and other diarylamino groups.

In general formula (1), preferable embodiments of L¹ include *p*-valent groups formed from aromatic compounds of formulae (3) to (6), preferably formulae (3), (4), and (6). The *p*-valent groups having a valence is formed by removing *p* hydrogen atoms from carbon atoms forming rings in formulae (3) to (6). When p is 2 or more, hydrogen atoms may be removed from the same ring or different rings.

In formulae (3) to (6), each X independently represents methine or nitrogen. Among Xs which form six-membered rings, 0 to 3 Xs are preferably nitrogen. More preferably, all Xs are methine. In formulae (4) and (6), Y represents oxygen or sulfur. In formula (5), r represents an integer of 0 to 2, and is preferably 0 or 1.

In formulae (3) to (6), each R' is independently hydrogen, a substituted or unsubstituted aromatic hydrocarbon group having 6 to 30 carbon atoms, a substituted or unsubstituted heteroaromatic ring group having 3 to 30 carbon atoms, an alkyl group having 1 to 12 carbon atoms, a diarylamino group having 12 to 44 carbon atoms, a cyano group, a nitro group, or a fluoro group. Each R' is preferably hydrogen, a substituted or unsubstituted aromatic hydrocarbon group having 6 to 18 carbon atoms, or a substituted or unsubstituted heteroaromatic ring group having 3 to 17 carbon atoms.

R' is the same as R in general formula (1) except that the heteroaromatic ring group comprises a carbazolyl group, and does not comprise a linked aromatic group.

In general formula (1), *p* represents an integer of 1 to 3. *p* is preferably 1 or 2, and more preferably 1.

In general formula (1), each *m* independently represents an integer of 2 to 4. *m* is preferably 2 or 3.

When *m* is 2 or more, there is a structure of a carbazolyl group directly bonded to a carbazolyl group. Preferably, in the formula, at least one binding structure represented by formula (d1) is present. It is preferable that all binding structures between carbazolyl groups be represented by only formula (d1) or only both of formula (c1) and formula (d1). It is more preferable that all binding structures between carbazolyl groups be represented by only both of formula (c1) and formula (d1). The carbazolyl group as used herein refers to a condensed ring of three rings in general formula (1). The total number of *m* (the total number of carbazolyl groups) is an integer of 2 to 12, preferably 2 to 9, and more preferably 2 to 6.

Preferable examples of the compound represented by general formula (1) are shown below, but the compound is not limited thereto.

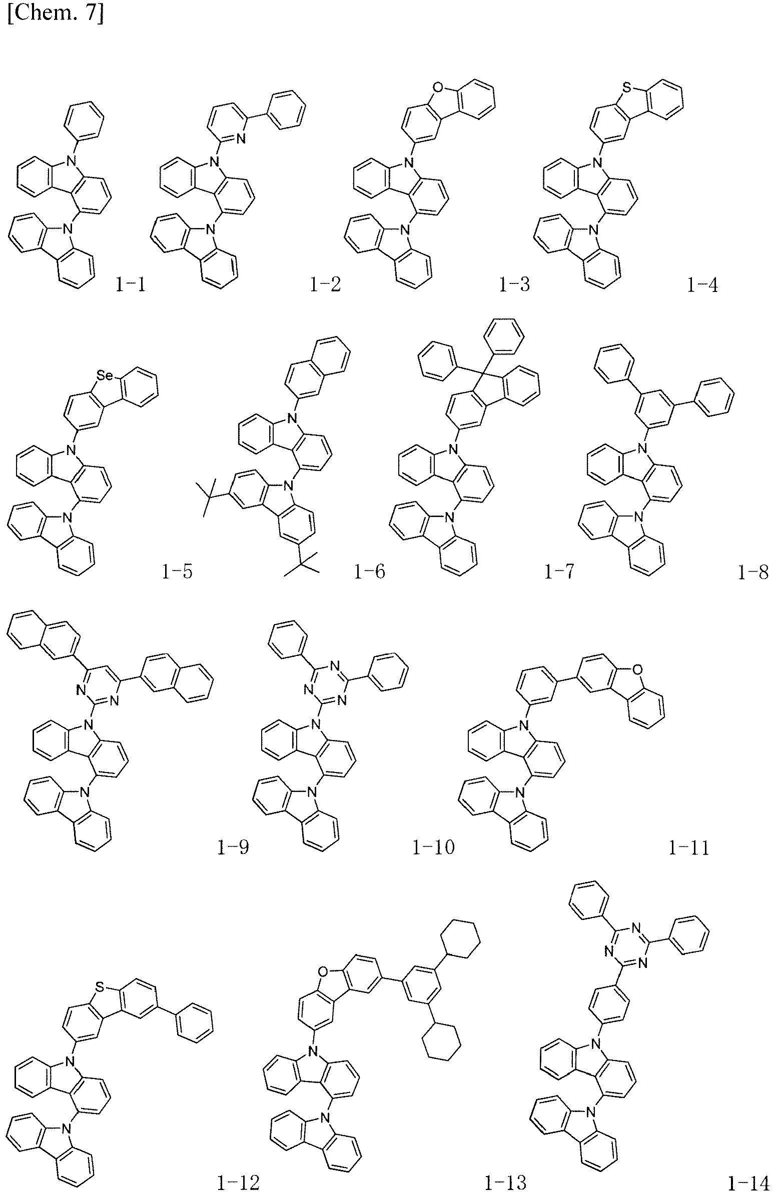

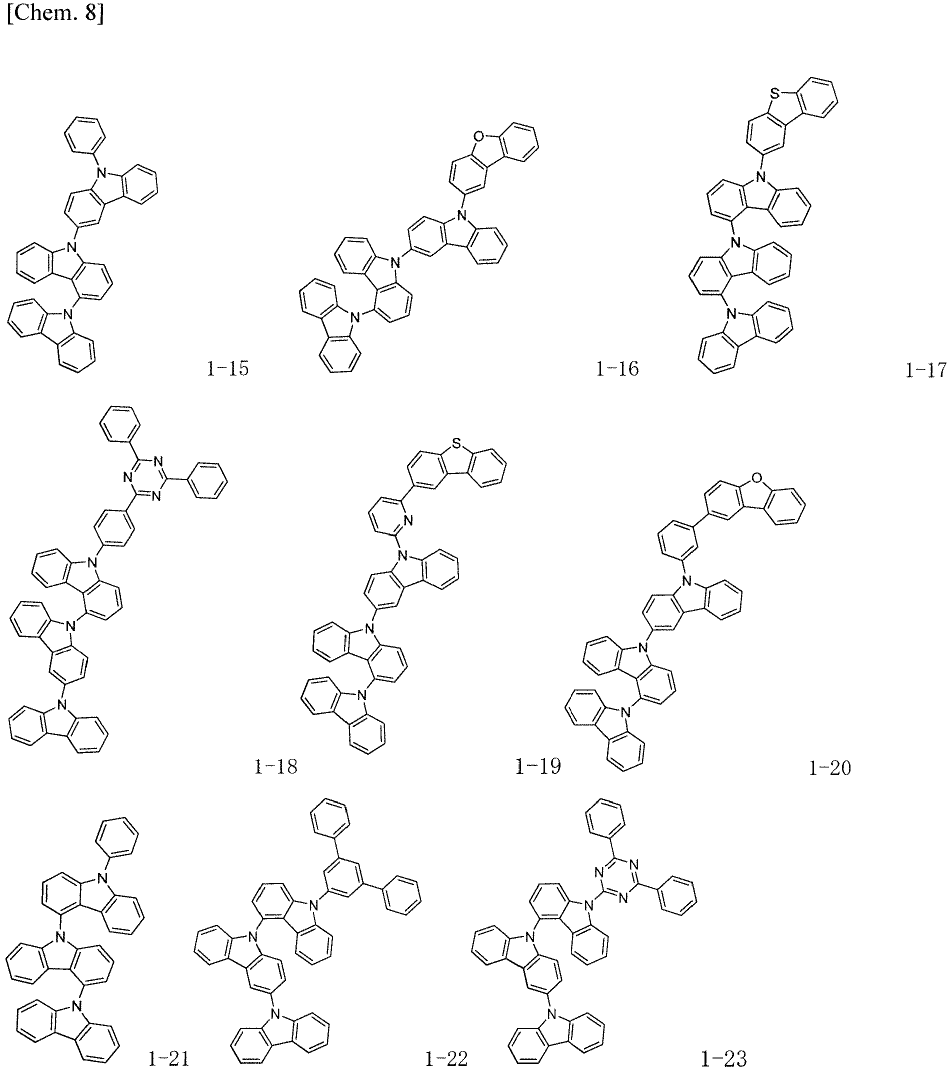

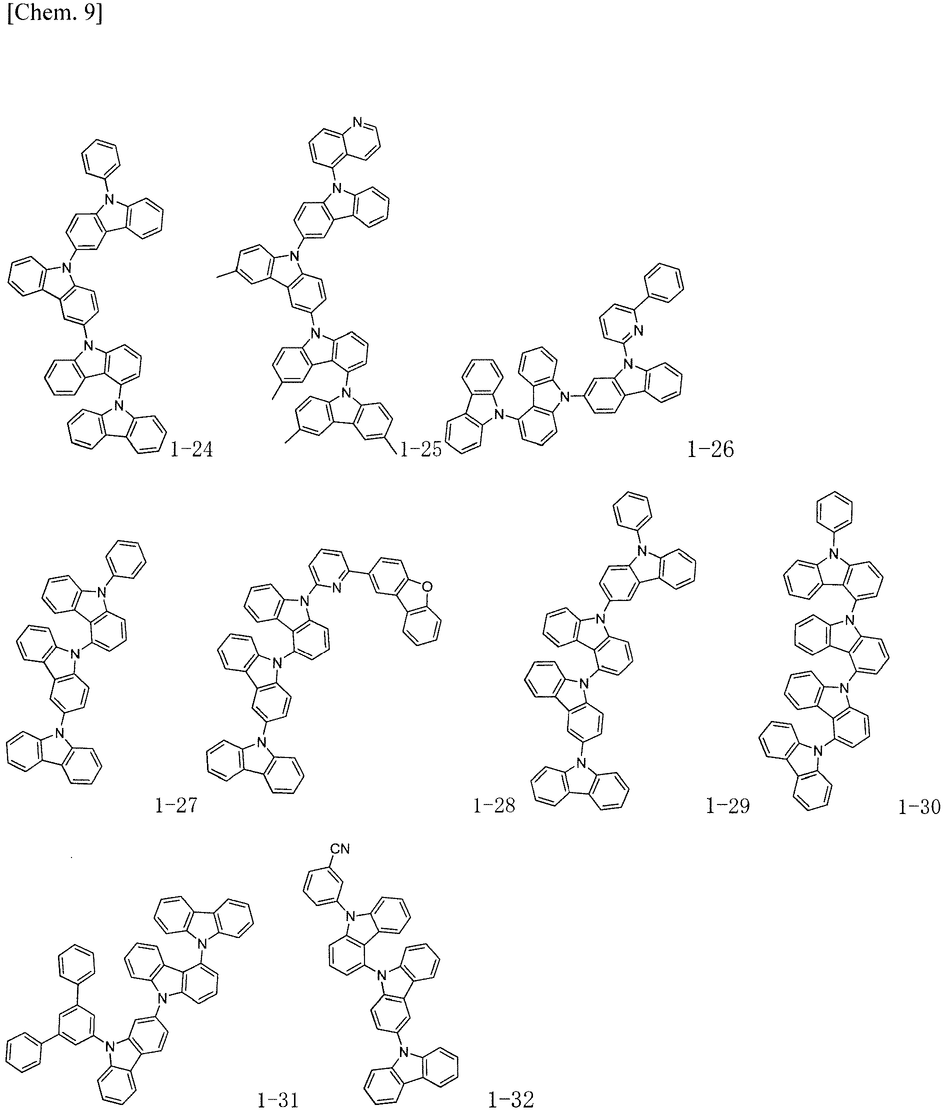

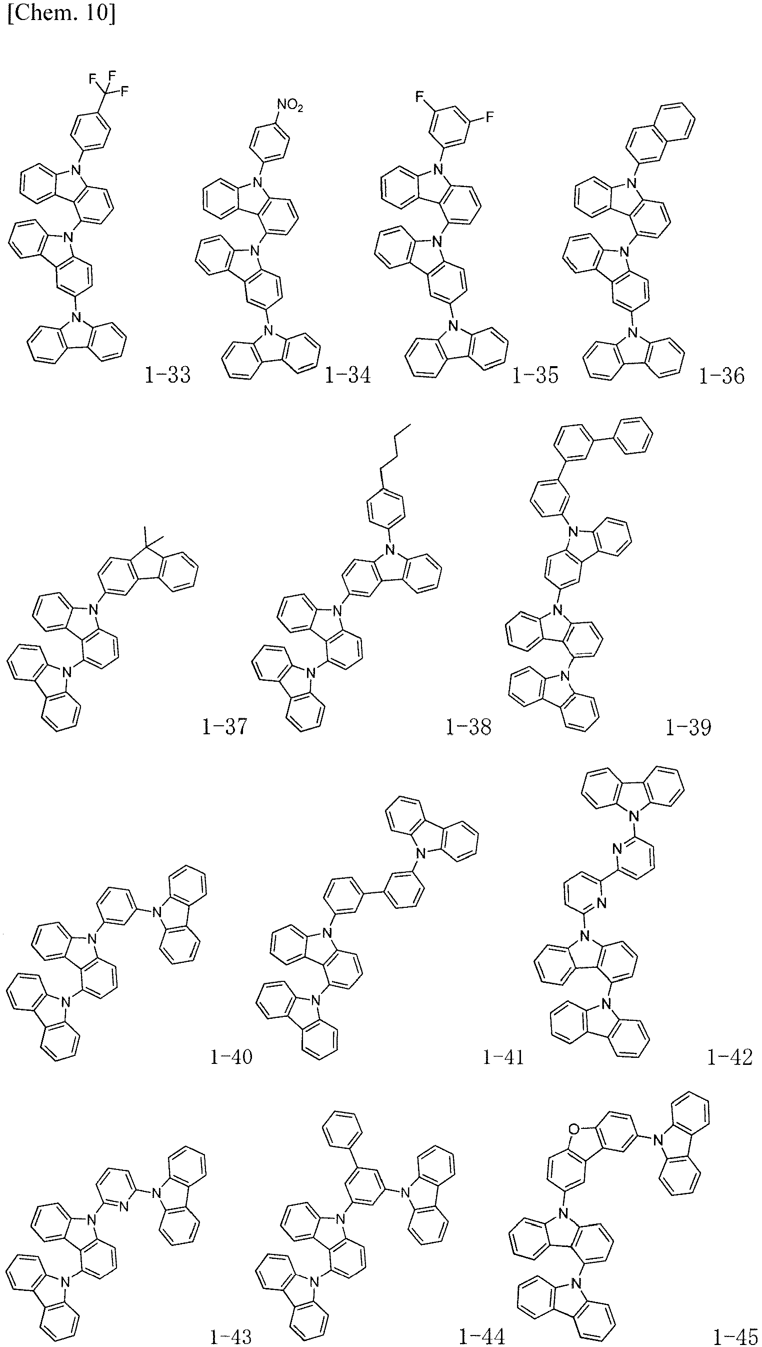

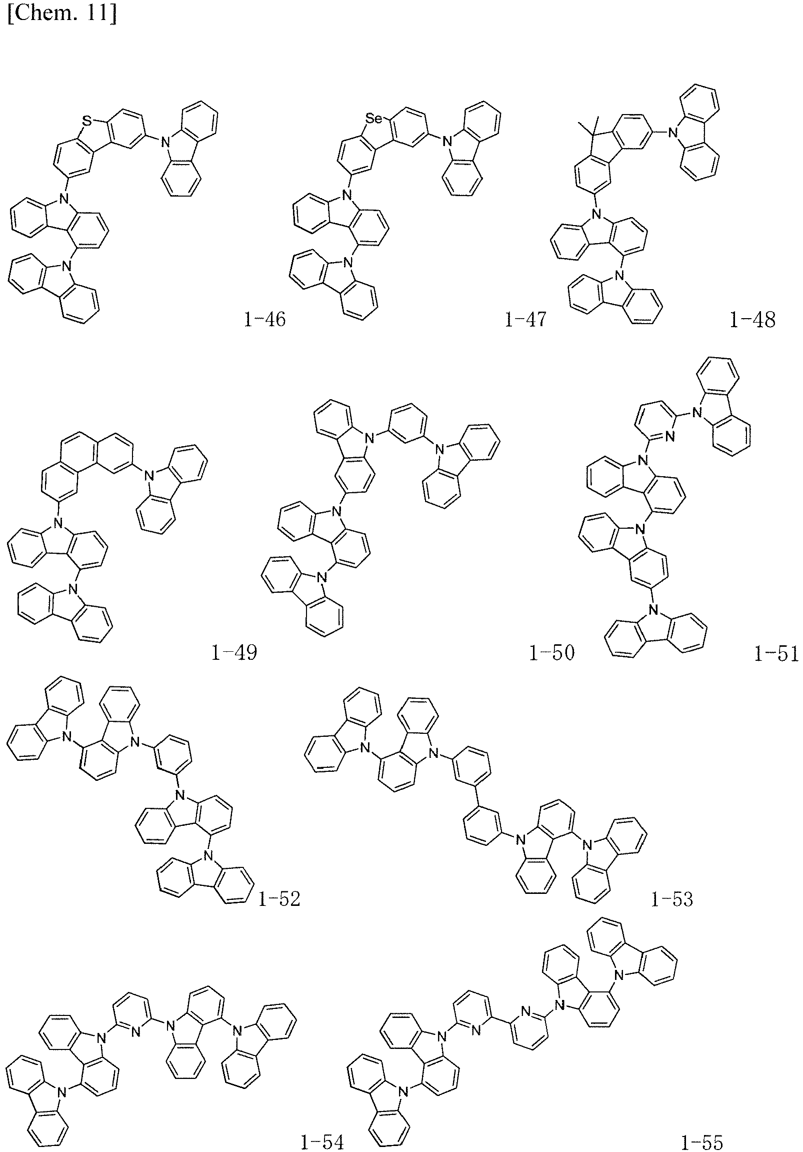

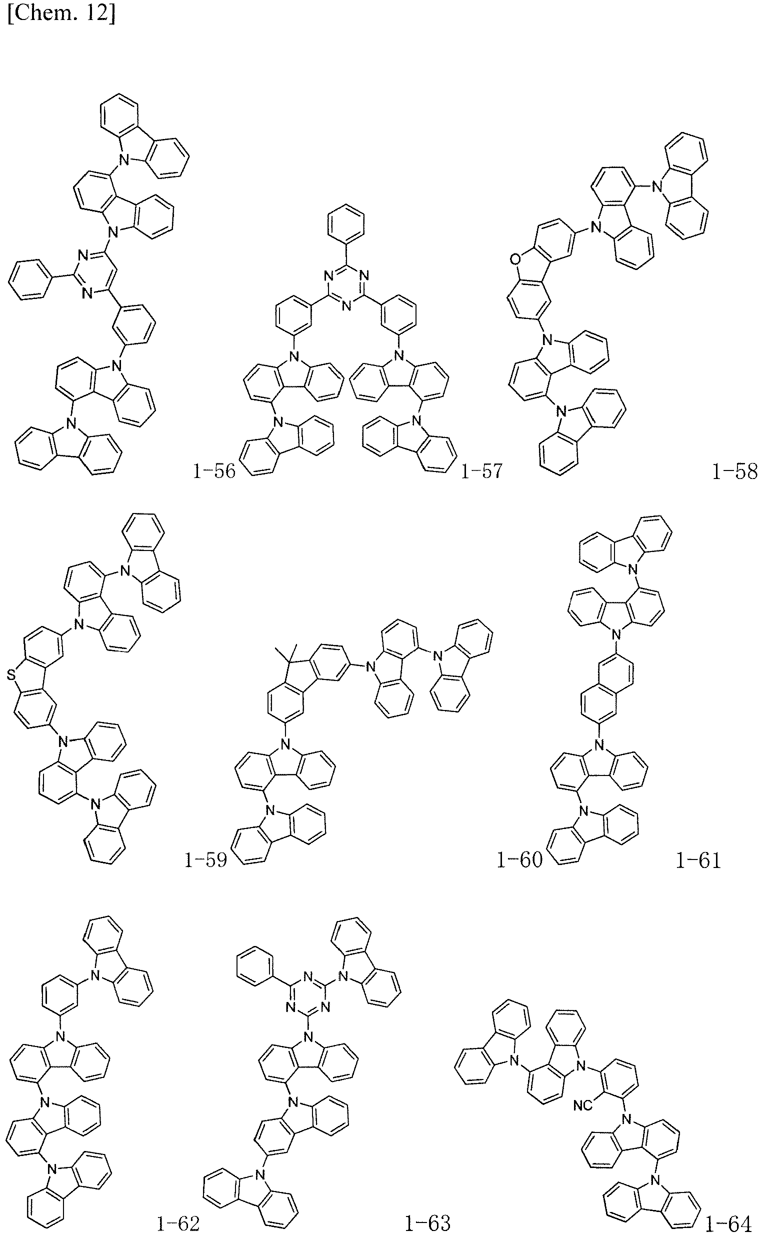

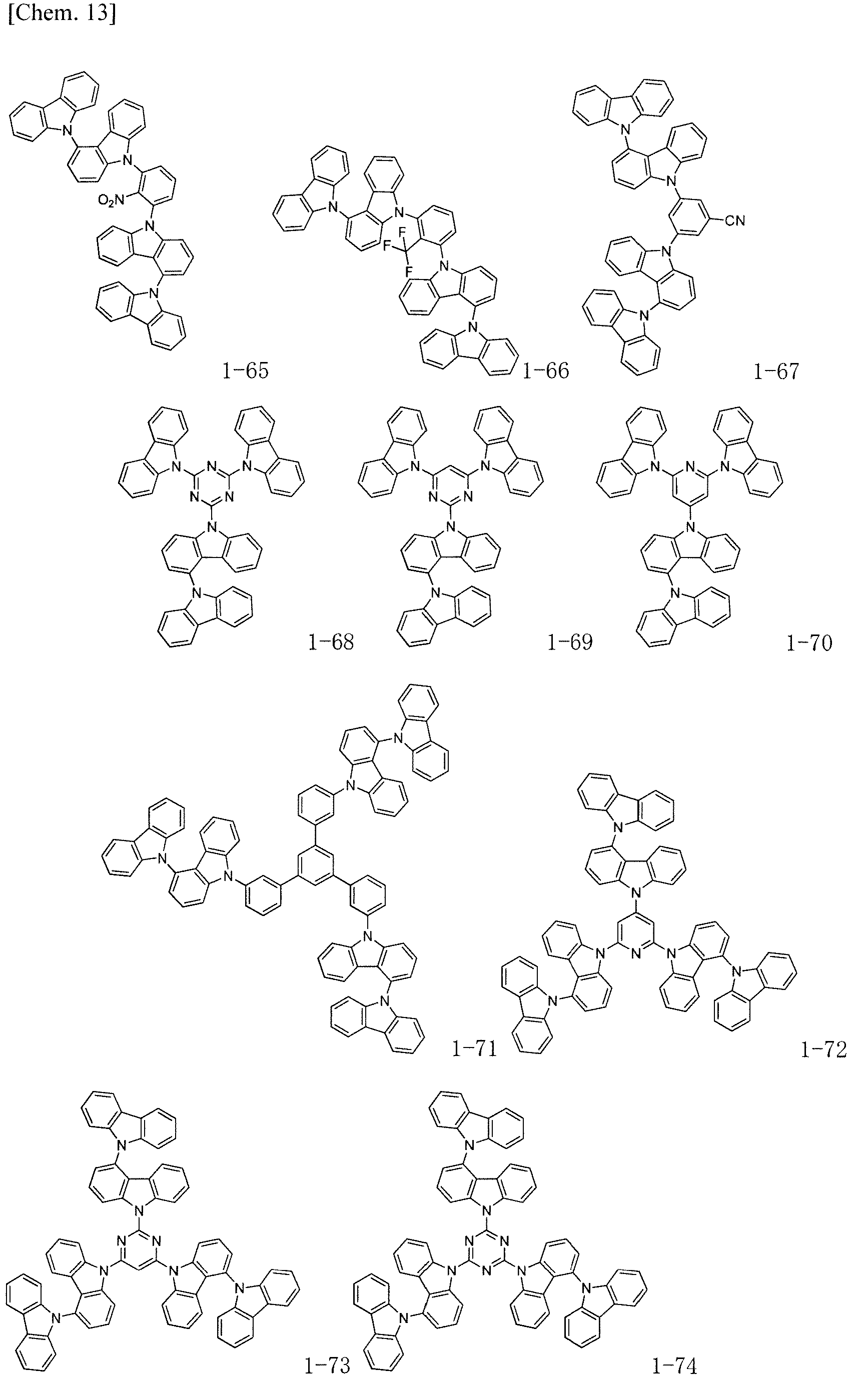

Next, the compound represented by general formula (2) (carborane compound) will be described. Ring A represents a divalent carborane group of C₂B₁₀H₁₀ represented by formula (a1) or formula (b1). A plurality of rings A in a molecule may be the same or different. It is preferable that all of rings A are carborane groups represented by formula (a1).

Two bonds of the divalent carborane group may start from C or B, but the bond to L² or L³ preferably starts from C.

*n* is a repeating number and represents an integer of 0 to 2. *n* is preferably 0 or 1, and more preferably 0.

*q* is a substitution number and represents an integer of 1 to 4. *q* is preferably an integer of 1 or 2, and is more preferably 1.

L² is a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 6 of the rings thereof. L² is preferably a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 4 of the aromatic rings thereof.

L³ is a single bond or a (*q* + 1)-valent group which is a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 6 of the aromatic rings thereof. L³ is preferably a single bond, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 4 of the aromatic rings thereof. However, when *q* = 1 and *n* = 1, L³ represents a single bond, an aromatic heterocyclic group, or a linked aromatic group comprising at least one aromatic heterocyclic group.

L⁴ independently represents a single bond or a divalent group. The divalent group is a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 6 of the aromatic rings thereof. L⁴ is preferably a single bond, a substituted or unsubstituted aromatic hydrocarbon group having 6 to 18 carbon atoms, a substituted or unsubstituted aromatic heterocyclic group having 3 to 17 carbon atoms, or substituted or unsubstituted linked aromatic group formed by linking 2 to 4 of the aromatic rings thereof.

When L², L³, and L⁴ in general formula (2) are an aromatic hydrocarbon group, an aromatic heterocyclic group, or a linked aromatic group formed by linking 2 or 6 of the aromatic rings, L², L³, or L⁴ is the same as described above regarding L¹ and R in general formula (1) except that a carbazolyl group is not excluded from the aromatic heterocyclic group. However, when *q* = 1 and *n* = 1, L³ represents a single bond, an aromatic heterocyclic group, or a linked aromatic group comprising at least one aromatic heterocyclic group.

When *n* = 0, it is preferable that L³ and L² be the same or aromatic rings of L³ and L², which bond to ring A, be the same. That the aromatic rings which bond to ring A are the same means that Ar² and Ar⁴, which bond to ring A, are the same when L³ represents Ar¹-Ar²- and L² represents -Ar³-Ar⁴-. Ar¹ to Ar⁴ are each an aromatic ring which may have a substituent. When n = 0, it is preferable that L² = L³-(H)q.

Preferable examples of the compound represented by general formula (2) are shown below, but the compound is not limited thereto.

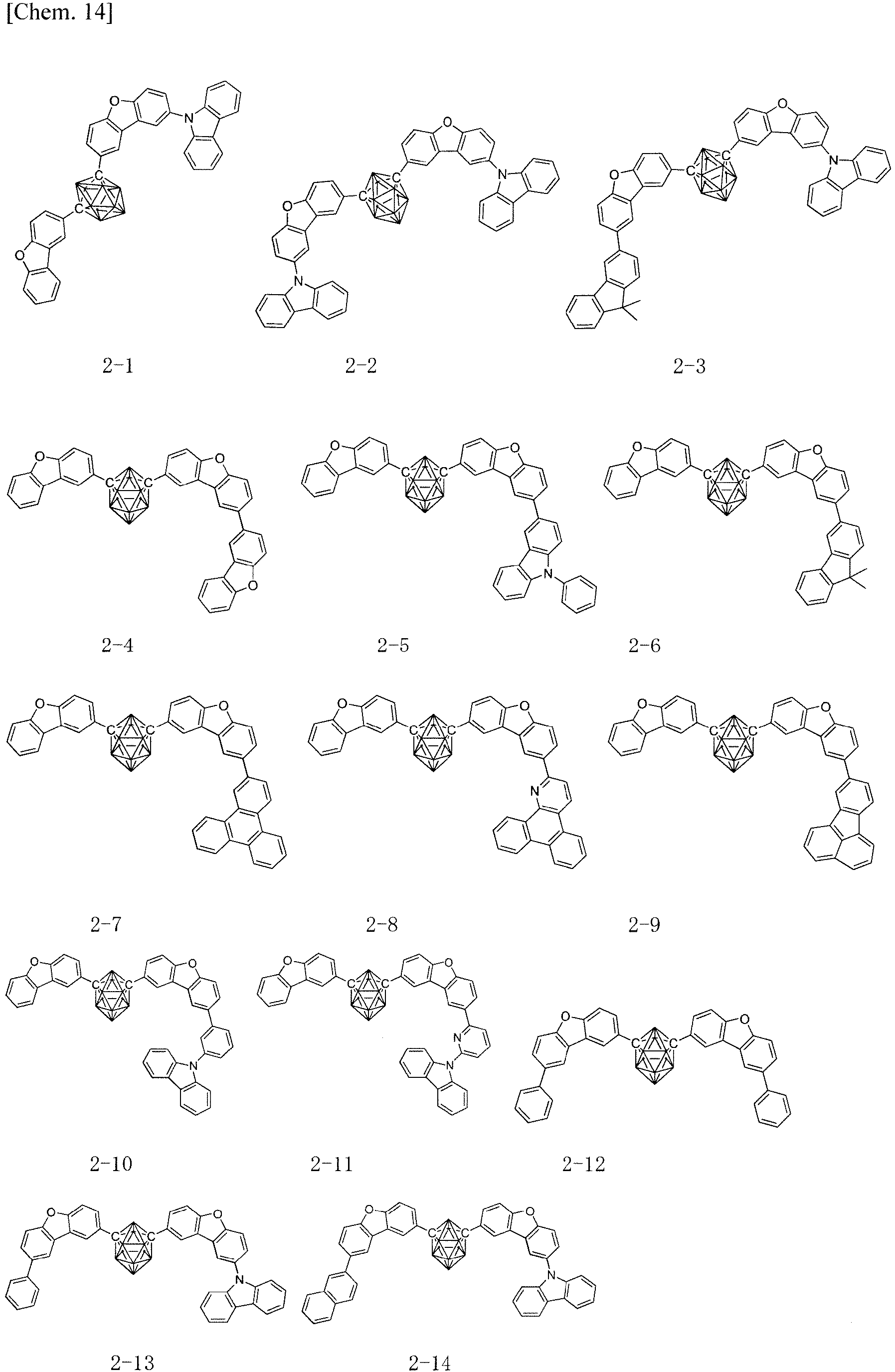

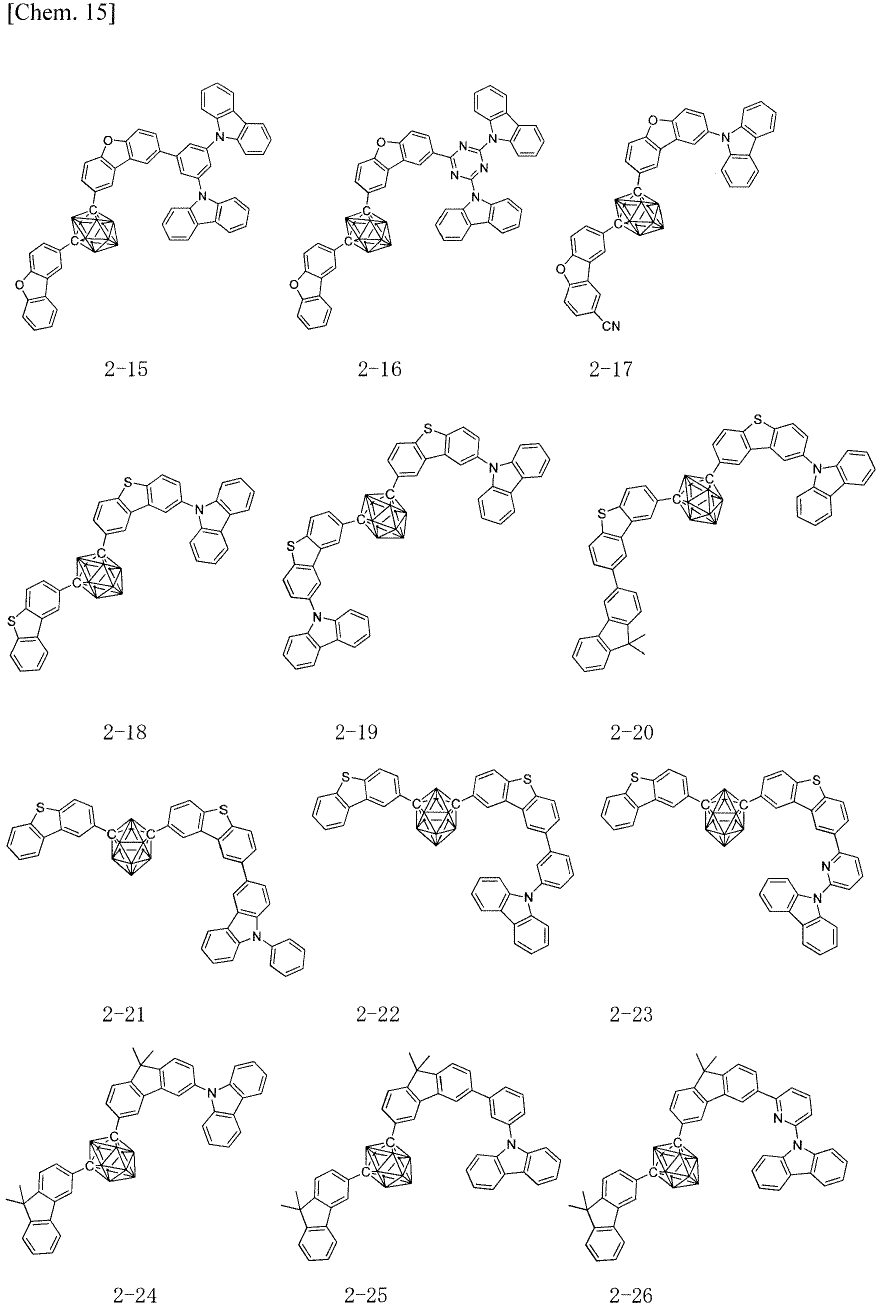

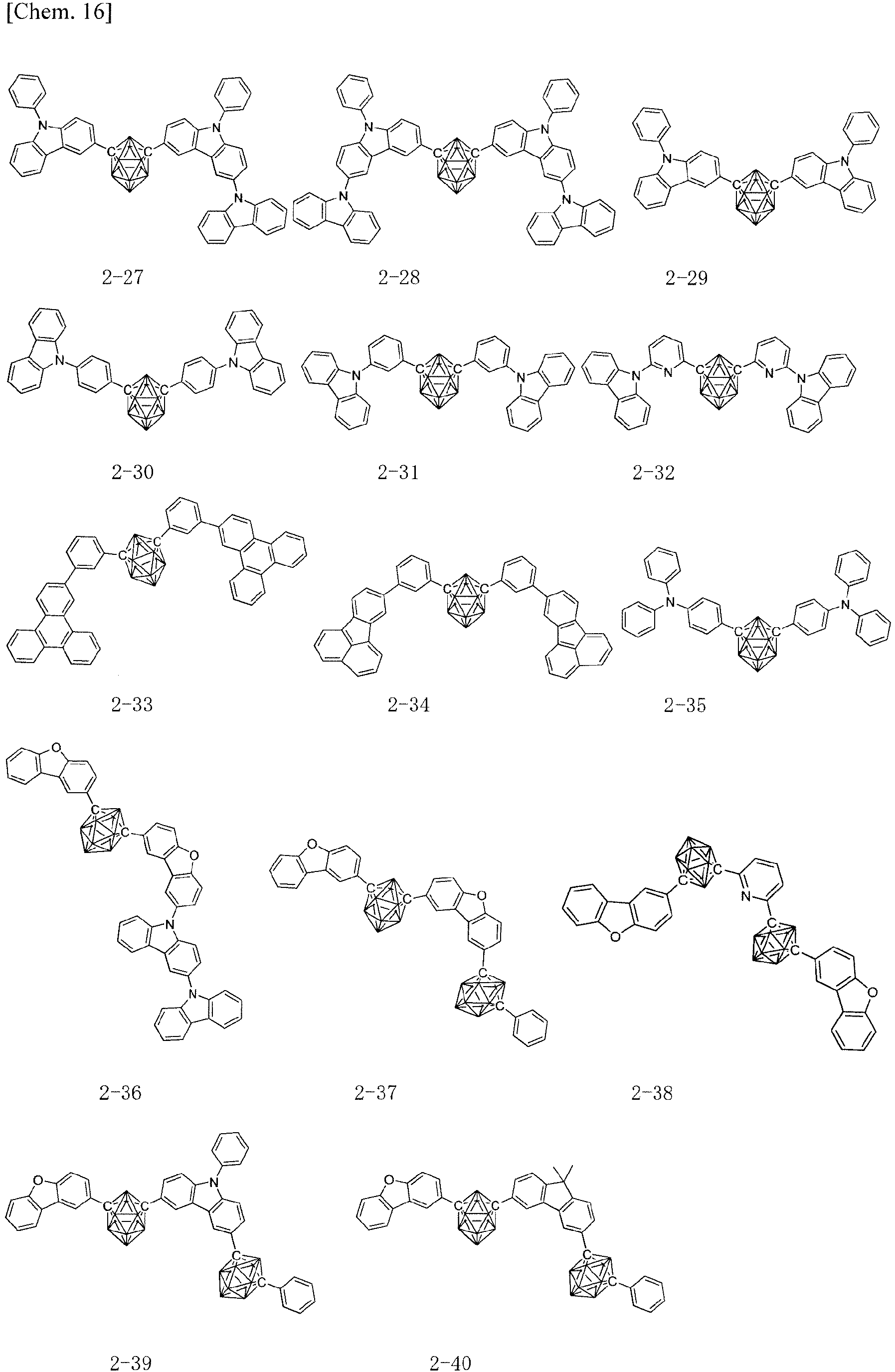

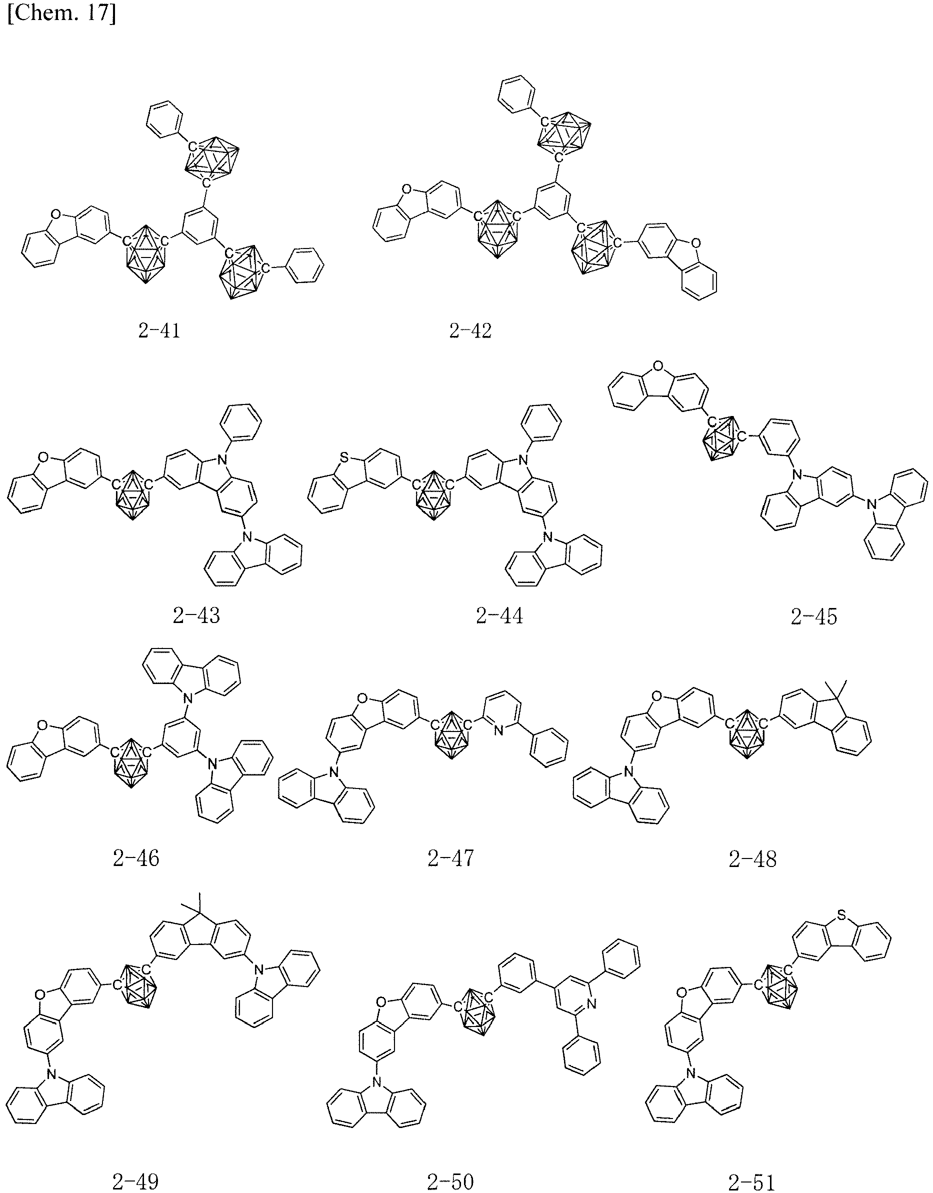

The organic EL device of the present invention comprises a mixture of a compound represented by general formula (1) and a compound represented by general formula (2) in at least one organic layer of the organic EL device. Since the mixture is excellent in charge transporting property, the mixture may be used in any organic layer. The mixture is preferably contained in a light-emitting layer, an electron-transporting layer, or a hole-blocking layer, and more preferably in a light-emitting layer.

When the mixture is used in a light-emitting layer, the mixture may be used as a luminescent dopant material, but is preferably used as a host material while another luminescent dopant material, a fluorescent dopant material, or a thermally-activated delayed fluorescent dopant material is used as the luminescent dopant material. In particular, an organometallic complex comprising at least one metal selected from ruthenium, rhodium, palladium, silver, rhenium, osmium, iridium, platinum and gold is a preferable embodiment of the luminescent dopant material.

The mixture may be mixed prior to forming the device and be deposited using a deposition source, or may be mixed by an operation, such as co-deposition using a plurality of deposition sources, at the time of forming the device.

The mixture may be used by forming a film on a substrate or the like using a wet process, such as spin coating or ink-jetting, without using a dry process with a deposition source.

Next, the structure of the organic EL device of the present invention will be described referring the drawing, but the structure of the organic EL device of the present invention is not limited to that illustrated in the drawing.

### (1) Configuration of Organic EL Device

FIG. 1 schematically shows the cross section of an example of an organic EL device generally used in this invention and **1** represents a substrate, **2** an anode, **3** a hole-injecting layer, **4** a hole-transporting layer, **5** a light-emitting layer, **6** an electron-transporting layer, **7** an electron-injecting layer, and **8** a cathode. The organic EL device of this invention comprises the anode, the light-emitting layer, the electron-transporting layer, and the cathode as essential layers and other layers may be provided as needed. Such other layers are, for example, a hole-injecting/transporting layer, an electron-blocking layer, and a hole-blocking layer, but are not limited thereto. The term "hole-injecting/transporting layer" means a hole-injecting layer and/or a hole-transporting layer.

### (2) Substrate

The substrate **1** serves as a support for an organic electroluminescent device and the materials useful therefor include a quartz plate, a glass plate, a metal sheet, a metal foil, a plastic film, and a plastic sheet. In particular, a glass plate and a flat, transparent sheet of synthetic resin such as polyester, polymethacrylate, polycarbonate, and polysulfone are preferred. In the case where a synthetic resin substrate is used, the gas barrier property of the resin needs to be taken into consideration. When the gas barrier property of the substrate is too low, the air passing through the substrate may undesirably deteriorate the organic electroluminescent device. One of the preferred methods for securing the gas barrier property is to provide a dense silicon oxide film or the like at least on one side of the synthetic resin substrate.

### (3) Anode

The anode **2** is provided on the substrate **1** and plays a role of injecting holes into the hole-transporting layer. The anode is usually constructed of a metal such as aluminum, gold, silver, nickel, palladium, and platinum, a metal oxide such as an oxide of indium and/or tin and an oxide of indium and/or zinc, a metal halide such as copper iodide, carbon black, and an electrically conductive polymer such as poly(3-methylthiophene), polypyrrole, and polyaniline. The anode is formed mostly by a process such as sputtering and vacuum deposition. In the case where silver or any other metal, copper iodide, carbon black, an electrically conductive metal oxide, or an electrically conductive polymer is available in fine particles, the anode can be formed by dispersing the particles in a solution of a suitable binder resin and coating the substrate with the dispersion. Further, in the case of an electrically conductive polymer, the anode can be formed as a thin film by performing electrolytic polymerization of the corresponding monomer directly on the substrate **1** or by coating the substrate with the polymer. The anode may also be formed by stacking different materials one upon another. The thickness of the anode varies with the requirement for transparency. In applications where transparency is required, it is desirable to control the transmission of visible light normally at 60% or more, preferably at 80% or more. In this case, the thickness becomes normally 5 to 1,000 nm, preferably 10 to 500 nm. In applications where opaqueness is accepted, the anode may be the same in transmission as the substrate. Furthermore, a different electrically conductive material can be stacked on the aforementioned anode.

### (4) Hole-transporting Layer

The hole-transporting layer **4** is provided on the anode **2** and the hole-injecting layer **3** may be disposed between the two. The condition that the material of choice for the hole-transporting layer must satisfy is an ability to inject holes from the anode at high efficiency and transport the injected holes efficiently. This makes it necessary for the material to satisfy the following requirements; low ionization potential, high transparency against visible light, high hole mobility, good stability, and low inclination to generate impurities that become traps of holes during fabrication and use. Further, since the hole-transporting layer is arranged in contact with the light-emitting layer **5,** the material for the hole-transporting layer must not lower the efficiency by quenching light emitted from the light-emitting layer or forming exciplexes with the light-emitting layer. Besides the aforementioned general requirements, heat resistance is required for applications such as vehicle-mounted display devices. Hence, the material desirably has a Tg of 85 °C or higher.

A mixture of general formula (1) and general formula (2) may be used as the hole-transporting material, or any of the compounds known thus far as hole-transporting materials may be used as such according to this invention. Examples include aromatic diamines containing two or more tertiary amines whose nitrogen atoms are substituted with two or more condensed aromatic rings, starburst aromatic amines such as 4,4',4"-tris(1-naphthylphenylamino)triphenylamine, an aromatic amine consisting of a tetramer of triphenylamine, and Spiro compounds such as 2,2',7,7'-tetrakis(diphenylamino)-9,9'-spirobifluorene. These compounds may be used alone or as a mixture if necessary.

In addition to the aforementioned compounds, examples of the hole-transporting materials include polymeric materials such as polyvinylcarbazole, polyvinyltriphenylamine, and polyaryleneethersulfone containing tetraphenylbenzidine.

When a coating process is used for forming the hole-transporting layer, a coating solution is prepared from one kind or two kinds or more of hole-transporting materials of choice and, if necessary, a binder resin which does not become a trap of holes and an additive such as an improver of coating properties are applied to the anode by a process such as spin coating, and dried to form the hole-transporting layer. Examples of the binder resin include polycarbonate, polyarylate, and polyester. As a binder resin lowers the hole mobility when added in a large amount, the binder is preferably added in a small amount, usually 50 wt% or less.

When a vacuum deposition process is used for forming the hole-transporting layer, the hole-transporting material of choice is introduced to a crucible placed in a vacuum container, the container is evacuated to 1×10⁻⁴ Pa or so by a suitable vacuum pump, the crucible is heated to evaporate the hole-transporting material, and the vapor is deposited on the substrate that has an anode formed thereon and is placed opposite the crucible to form the hole-transporting layer. The thickness of the hole-transporting layer is normally 1 to 300 nm, preferably 5 to 100 nm. The vacuum deposition process is generally used to form such a thin film uniformly.

### (5) Hole-injecting Layer

For the purpose of still further enhancing the hole-injecting efficiency and improving the adhesive strength of the organic layer to the anode as a whole, the hole-injecting layer **3** is disposed between the hole-transporting layer **4** and the anode **2.** Disposition of the hole-injecting layer produces an effect of lowering the driving voltage of the device in the initial period and, at the same time, suppressing a rise in voltage during continuous driving of the device at constant current density. The hole-injecting material of choice must satisfy the following requirements; it is formable into a thin film that is uniform in quality and makes good contact with the anode, and it is thermally stable. Namely, the material is required to have a high glass transition which is 100 °C or above. Further, the material is required to have a low ionization potential to facilitate injection of holes from the anode and exhibit high hole mobility.

For this purpose, a mixture of general formula (1) and general formula (2) may be used or any of the compounds known thus far, such as phthalocyanine compounds such as copper phthalocyanine; organic compounds such as polyaniline and polythiophene; sputtered carbon membranes; metal oxides such as vanadium oxide, ruthenium oxide, and molybdenum oxide; and p-type organic compounds such as 1,4,5,8-naphthalenetetracarboxylic dianhydride (NTCDA) and hexanitrilehexaazatriphenylene (HAT), may be used alone or mixed as needed. The hole-injecting layer can also be formed as a thin film, like the hole-transporting layer, and in the case where the material of choice is an inorganic compound, a process such as sputtering, electron beam deposition, or plasma CVD can be used. The thickness of the hole-injecting layer formed as described above is normally 1 to 300 nm, preferably 5 to 100 nm.

### (6) Light-emitting Layer

The light-emitting layer **5** is provided on the hole-transporting layer **4.** The light-emitting layer may be composed of a single light-emitting layer or it may be constructed by stacking a plurality of light-emitting layers one upon another. The light-emitting layer is composed of a host material and a luminescent dopant. The luminescent dopant may be a fluorescent material, a delayed fluorescent material, or a phosphorescent material. A mixture of compounds of general formula (1) and general formula (2) may be used as the luminescent dopant, but is preferably used as the host material.

In the case of the fluorescent organic EL device, materials to be added to the host materials include derivatives of condensed ring compounds such as perylene and rubrene, quinacridone derivatives, Phenoxazone 660, DCM1, perinone, coumarin derivatives, pyrromethene (diazaindacene) derivatives, and cyanine dyes.

In the case of the phosphorescent organic EL device, examples of the delayed fluorescent material in the light-emitting layer include carborane derivatives, tin complexes, indolocarbazole derivatives, copper complexes, and carbazole derivatives. Specific examples include the compounds described in the following Non-Patent Literature and Patent Literature, but the delayed fluorescent material is not limited thereto.

1) Adv. Mater. 2009, 21, 4802-4806
2) Appl. Phys. Lett. 98, 083302 (2011)
3) JP 2011-213643 A
4) J. Am. Chem. Soc. 2012, 134, 14706-14709

Specific examples of the delayed luminescent material are described below, but the delayed luminescent material is not limited thereto.

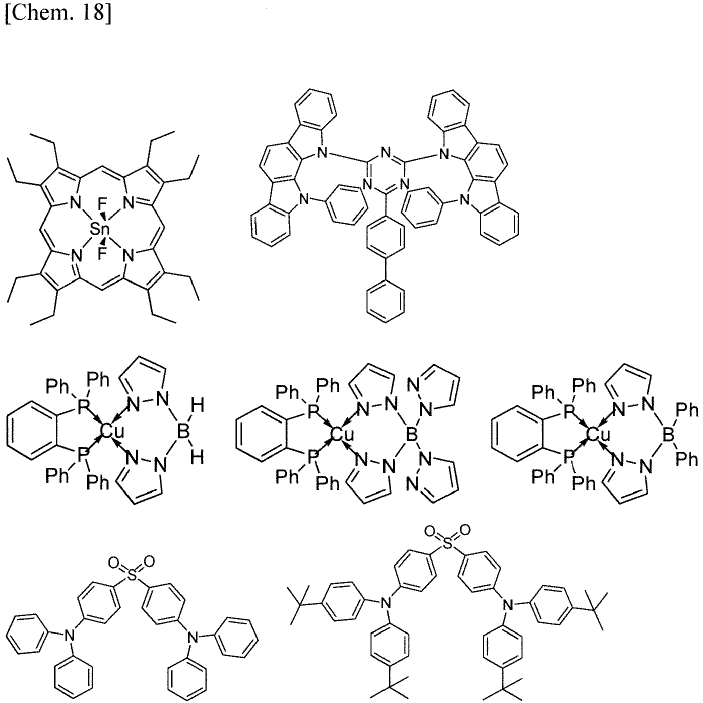

When the delayed fluorescent material is used as the delayed fluorescence dopant and a host material is contained therein, the content of the delayed fluorescence dopant contained in the light-emitting layer is 0.01 to 50 wt%, preferably 0.1 to 20 wt%, and more preferably 0.01 to 10%.

In the case of a phosphorescent organic EL device, an organometallic complex comprising at least one metal selected from ruthenium, rhodium, palladium, silver, rhenium, osmium, iridium, platinum and gold is preferable as the phosphorescent dopant. Specific examples of the phosphorescent dopant are described in the Patent Literature below, but the phosphorescent dopant is not limited thereto. As the host material, a mixture comprising a compound represented by general formula (1) and a compound represented by general formula (2) is excellent.

WO 2009-073245 A1, WO 2009-046266 A1, WO 2007-095118 A1, WO 2008-156879 A1, WO 2008-140657 A1, US 2008-261076 A1, JP 2008-542203 A, WO 2008-054584 A1, JP 2008-505925 A, JP 2007-522126 A, JP 2004-506305 A, JP 2006-513278 A, JP 2006-50596 A, WO 2006-046980 A1, WO 2005-113704 A1, US 2005-260449 A1, US 2005-2260448 A1, US 2005-214576 A1, WO 2005-076380 A1, etc.

Preferred examples of the phosphorescent light-emitting dopant include complexes such as Ir(PPy)₃, complexes such as Ir(bt)2•acac3, and complexes such as PtOEt3, the complexes each having a noble metal device such as Ir as a central metal. Specific examples of those complexes are shown below, but the phosphorescent light-emitting dopant is not limited to the compounds described below.

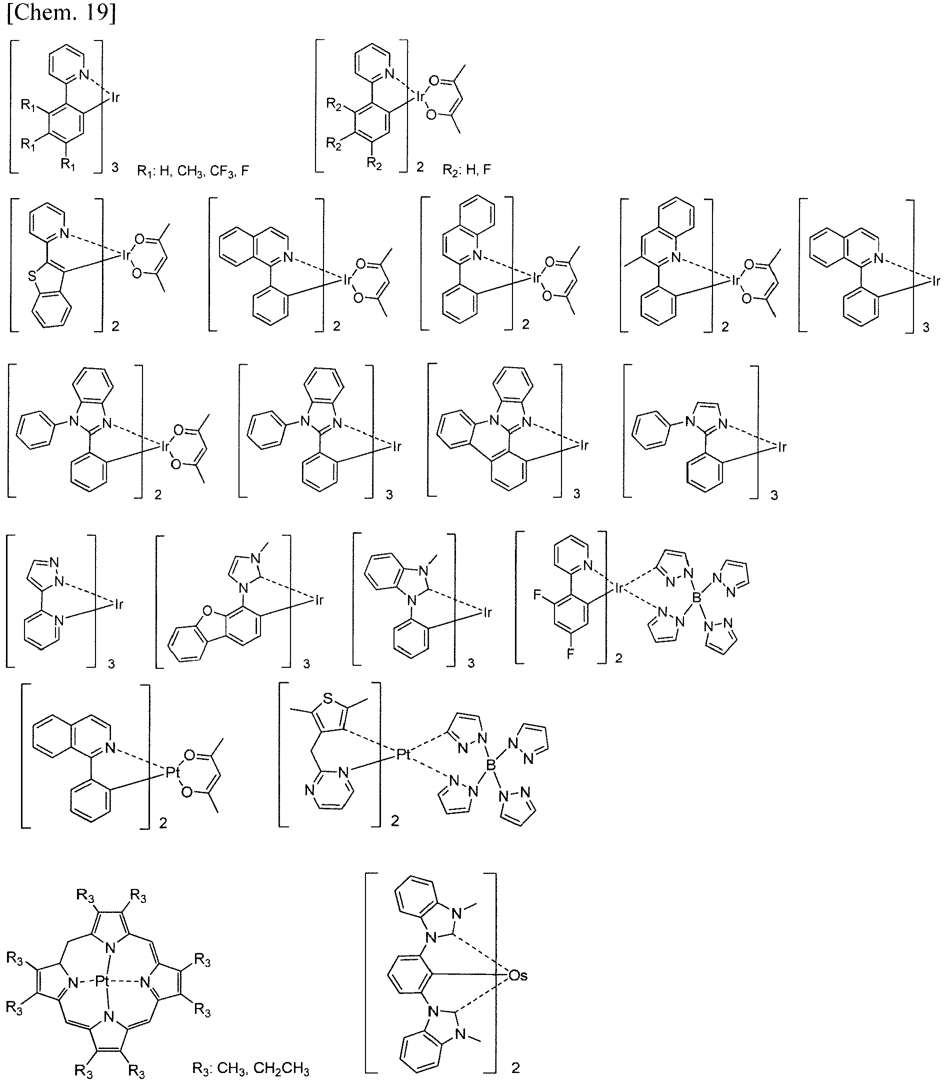

It is preferred that the content of the phosphorescent light-emitting dopant in the light-emitting layer be in the range of from 2 to 40 wt%, preferably from 5 to 30 wt%.

The thickness of the light-emitting layer, which is not particularly limited, is typically from 1 to 300 nm, preferably from 5 to 100 nm, and a thin film serving as the layer is formed by the same method as that for the hole-transporting layer.

### -Blocking Layer-

The blocking layer is capable of blocking electric charge (electrons or holes) and/or excitons present in the light-emitting layer from diffusing to the outside of the light-emitting layer. The electron-blocking layer may be disposed between the light-emitting layer and the hole-transporting layer and block electrons from passing through the light-emitting layer toward the hole-transporting layer. Similarly, the hole-blocking layer may be disposed between the light-emitting layer and the electron-transporting layer and block holes from passing through the light-emitting layer toward the electron-transporting layer. The blocking layer may also be used to block excitons from diffusing to the outside of the light-emitting layer. That is, the electron-blocking layer and the hole-blocking layer may respectively have the function of an exciton-blocking layer. The term "electron-blocking layer" or "hole-blocking layer" as used herein means that a layer comprises one layer by itself having the function of a charge (electron or hole) blocking layer and an exciton-blocking layer.

### -Hole-blocking layer-

The hole-blocking layer has the function of an electron transporting layer in a broad sense. The hole-blocking layer has a function of inhibiting holes from reaching the electron transporting layer while transporting electrons, and thereby enhances the recombination probability of electrons and holes in the light-emitting layer.

As the material for the hole-blocking layer, a mixture of general formula (1) and general formula (2) is preferably used, and the materials for the electron-transporting layer described later may be used. The thickness of the hole-blocking layer of the present invention is preferably 3 to 100 nm and more preferably 5 to 30 nm.

### -Electron-blocking layer-

The electron-blocking layer has the function of transporting holes in a broad sense. The electron-blocking layer has a function of inhibiting electrons from reaching the hole transporting layer while transporting holes, and thereby enhances the recombination probability of electrons and holes in the light-emitting layer.

As the material for the electron-blocking layer, a mixture of general formula (1) and general formula (2) is preferably used, and the materials for the hole-transporting layer described later may be used. The thickness of the electron-blocking layer of the present invention is preferably 3 to 100 nm and more preferably 5 to 30 nm.

### -Exciton-blocking layer-

The exciton-blocking layer is a layer for inhibiting excitons generated through the recombination of holes and electrons in the light-emitting layer from being diffused to the charge transporting layer, and inserting the layer enables effective confinement of excitons in the light-emitting layer, and thereby enhances the luminous efficacy of the device. The exciton-blocking layer may be inserted adjacent to the light-emitting layer on any of the side of the anode and the side of the cathode, and on both the sides. Specifically, in the case where the exciton-blocking layer is present on the side of the anode, the layer may be inserted between the hole transporting layer and the light-emitting layer and adjacent to the light-emitting layer, and in the case where the layer is inserted on the side of the cathode, the layer may be inserted between the light-emitting layer and the cathode and adjacent to the light-emitting layer. Between the anode and the exciton-blocking layer that is adjacent to the light-emitting layer on the side of the anode, a hole injection layer, an electron barrier layer and the like may be provided, and between the cathode and the exciton-blocking layer that is adjacent to the light-emitting layer on the side of the cathode, an electron injection layer, an electron transporting layer, a hole barrier layer and the like may be provided.

As the material for the exciton-blocking layer, a mixture of general formula (1) and general formula (2) is preferably used, and any commonly used material may be used.

Examples of known exciton-blocking materials that may be used herein include 1,3-dicarbazolylbenzene (mCP) and bis(2-methyl-8-quinolinolato)(4-phenylphenolato)aluminum (III) (BAlq).

### (7) Electron-Transporting Layer

For the purpose of enhancing the luminous efficiency of the device further still, the electron-transporting layer **6** is disposed between the light-emitting layer **5** and the cathode **8.** As the electron-transporting layer, an electron-transporting material which can smoothly inject electrons from the cathode is preferable. A mixture of general formula (1) and general formula (2) may be used and any commonly used materials may be used. Examples of the electron-transporting material which satisfies such limitations include metal complexes such as Alq3, 10-hydroxybenzo[h]quinoline metal complexes, oxadiazole derivatives, distyrylbiphenyl derivatives, silole derivatives, 3- or 5-hydroxyflavone metal complexes, benzoxazole metal complexes, benzothiazole metal complexes, trisbenzimidazolybenzene, quinoxaline compounds, phenanthroline derivatives, 2-t-butyl-9,10-N,N'-dicyanoanthraquinonediimine, n-type hydrogenated amorphous silicon carbide, n-type zinc sulfide, and n-type zinc selenide.

The thickness of the electron-transporting layer is typically 1 to 300 nm and is preferably 5 to 100 nm. The electron-transporting layer is formed on the light-emitting layer by coating or vacuum deposition as in the case of the hole-transporting layer. The vacuum deposition process is usually employed.

### (8) Cathode

The cathode **8** plays a role of injecting electrons into the electron-transporting layer **6.** The materials useful for the cathode may be the same as the aforementioned materials for the anode **2.** However, a metal having a low work function is desirable for efficient injection of electrons and a metal such as tin, magnesium, indium, calcium, aluminum, and silver or any of alloys thereof may be used. Specific examples are electrodes made from alloys having a low work function such as magnesium-silver alloys, magnesium-indium alloys, and aluminum-lithium alloys.

The thickness of the cathode is usually the same as that of the anode. For the purpose of protecting the cathode made from a metal having a low work function, covering the cathode with a metal of high work function that is stable against the air improves the stability of the device. A metal such as aluminum, silver, copper, nickel, chromium, gold, and platinum is used for this purpose.

Further, disposition of the electron-injecting layer **7** in the form of an ultrathin insulating film (0.1 to 5 nm) of LiF, MgF₂, Li₂O, or the like between the cathode **8** and the electron-transporting layer **6** is also an effective method for enhancing the efficiency of the device.

It is possible to fabricate a device with a structure that is the reverse of the structure shown in FIG. 1; that is, the device is fabricated by stacking, on the substrate **1,** the cathode **8,** the electron-injecting layer **7,** the electron-transporting layer **6,** the light-emitting layer **5,** the hole-transporting layer **4,** the hole-injecting layer **3,** and the anode **2** one upon another in this order. As described earlier, it is also possible to dispose the organic EL device of the present invention between two substrates at least one of which is highly transparent. In this case of the reverse structure, it is also possible to add or omit a layer or layers as needed.

The organic EL device of this invention is applicable to a single device, a device with its structure arranged in array, or a device in which the anode and the cathode are arranged in an X-Y matrix. According to this invention, a combination of the first electron-transporting layer containing a compound of specified skeleton with the second electron-transporting layer containing an existing electron-transporting material other than the compound of specified skeleton or a material comparable to the existing material provides an organic EL device that can perform at enhanced luminous efficiency with markedly improved driving stability even at low voltage. The organic EL device thus obtained displays excellent performance when applied to full-color or multicolor panels.

This invention will be described in more detail below with reference to the Examples, but will not be limited thereto. This invention can be reduced to practice in various modes unless such practice exceeds the substance of this invention. The first host and compound A refer to a compound represented by represented by general formula (1), and the second host and compound B refer to a compound represented by represented by general formula (2).

### Example 1

A thin film was laminated by a vacuum deposition method at a degree of vacuum of 2.0× 10⁻⁵ Pa on a glass substrate having formed thereon an anode comprising indium tin oxide (ITO) having a thickness of 70 nm. First, copper phthalocyanine (CuPC) was formed into a layer having a thickness of 30 nm to serve as a hole-injecting layer on the ITO. Next, 4,4-bis[N-(1-naphthyl)-N-phenylamino]biphenyl (NPB) was formed into a layer having a thickness of 15 nm to serve as a hole-transporting layer. Next, compound 1-2 serving as a first host and compound 2-1 serving as a second host for a light-emitting layer and an iridium complex [iridium(III) bis(4,6-di-fluorophenyl)-pyridinato-N,C2']picolinate] (FIrpic) serving as a blue phosphorescent material as a light-emitting layer guest were co-deposited from different deposition sources onto the hole-transporting layer to form a light-emitting layer having a thickness of 30 nm. At this time, a vapor deposition rate ratio among the first host, the second host, and FIrpic was 47:47:6 (by weight). Next, Alq₃ was formed into a layer having a thickness of 25 nm to serve as an electron-transporting layer. Further, lithium fluoride (LiF) was formed into a layer having a thickness of 1.0 nm to serve as an electron-injecting layer on the electron-transporting layer. Finally, aluminum (Al) was formed into a layer having a thickness of 70 nm to serve as an electrode on the electron-injecting layer. The resulting organic EL device has a layer construction comprising the electron-injecting layer added between the cathode and the electron-transporting layer in the organic EL device illustrated in FIG. 1.

An external power source was connected to the resultant organic EL device and a DC voltage was applied to the device. As a result, an emission spectrum having a local maximum wavelength of 475 nm was observed and it was found that light emission from FIrpic was obtained. Table 1 shows the properties of the produced organic EL device.

### Examples 2 to 21

Organic EL devices were each produced in the same manner as in Example 1 except that in Example 1, a compound shown in Table 1 was used as the first host of the light-emitting layer (Examples 2 to 7).

Organic EL devices were each produced in the same manner as in Examples 1 to 7 except that Compound 2-18 or 2-29 shown in Table 1 was used as the second host of the light-emitting layer (Examples 8 to 21).

An external power source was connected to each of the resultant organic EL devices and a DC voltage was applied to the device. As a result, an emission spectrum having a local maximum wavelength of 475 nm was observed for each of the organic EL devices and it was found that light emission from FIrpic was obtained. Table 1 shows the properties of each of the produced organic EL devices.

### Comparative Examples 1 to 10

Organic EL devices were each produced in the same manner as in Example 1 except that in Example 1, a compound shown in Table 1 was used alone as the light-emitting layer host. The host amount was set to the same amount as the total of the first host and second host in Example 1, and the guest amount was the same. A power source was connected to each of the resultant organic EL devices and a DC voltage was applied to the device. As a result, an emission spectrum having a local maximum wavelength of 475 nm was observed for each of the organic EL devices and it was found that light emission from FIrpic was obtained. Table 2 shows the properties of the produced organic EL devices.

In Tables 1 and 2, the luminance, the voltage, and the luminous efficacy are values at a driving current of 2.5 mA/cm², and the luminance half-time is a value at an initial luminance of 1,000 cd/m². Compound No.is the number attached to the chemical formulae.

**[Table 1]**

| Example | 1st host compound No. | 2nd host compound No. | Luminance (cd/m²) | Voltage (V) | Luminous efficiency (lm/W) | Luminance half-time (h) |
|---|---|---|---|---|---|---|
| 1 | 1-2 | 2-1 | 610 | 5.3 | 14.5 | 1800 |
| 2 | 1-8 | | 610 | 5.4 | 14.3 | 1800 |
| 3 | 1-11 | | 610 | 5.3 | 14.5 | 1800 |
| 4 | 1-15 | | 610 | 5.4 | 14.2 | 3000 |
| 5 | 1-44 | | 620 | 5.5 | 14.1 | 2100 |
| 6 | 1-45 | | 610 | 5.6 | 13.8 | 2700 |
| 7 | 1-68 | | 620 | 6.3 | 12.3 | 2100 |
| 8 | 1-2 | 2-18 | 610 | 5.7 | 13.4 | 1620 |
| 9 | 1-8 | | 610 | 5.8 | 13.3 | 1620 |
| 10 | 1-11 | | 600 | 5.7 | 13.2 | 1620 |
| 11 | 1-15 | | 610 | 5.8 | 13.1 | 2700 |
| 12 | 1-44 | | 610 | 5.8 | 13.3 | 1890 |
| 13 | 1-45 | | 600 | 6.1 | 12.4 | 2430 |
| 14 | 1-68 | | 600 | 5.9 | 12.9 | 1890 |
| 15 | 1-2 | 2-29 | 610 | 5.5 | 14.0 | 1440 |
| 16 | 1-8 | | 610 | 6.3 | 12.2 | 1440 |
| 17 | 1-11 | | 610 | 5.4 | 14.1 | 1440 |
| 18 | 1-15 | | 610 | 5.9 | 12.9 | 2400 |
| 19 | 1-44 | | 610 | 6.2 | 12.3 | 1680 |
| 20 | 1-45 | | 610 | 6.1 | 12.6 | 2160 |
| 21 | 1-68 | | 610 | 6.1 | 12.7 | 1680 |

**[Table 2]**

| Comp. Ex. | 1st host compound No. | 2nd host compound No. | Luminance (cd/m²) | Voltage (V) | Luminous efficiency (lm/W) | Luminance half-time (h) |
|---|---|---|---|---|---|---|
| 1 | 1-2 | - | 410 | 6.2 | 8.3 | 430 |
| 2 | 1-8 | - | 410 | 7.1 | 7.2 | 430 |
| 3 | 1-11 | - | 410 | 6.0 | 8.6 | 430 |
| 4 | 1-15 | - | 410 | 7.0 | 7.4 | 630 |
| 5 | 1-44 | - | 420 | 6.9 | 7.6 | 490 |
| 6 | 1-45 | - | 410 | 7.4 | 6.9 | 630 |
| 7 | 1-68 | - | 420 | 8.3 | 6.4 | 490 |
| 8 | - | 2-1 | 410 | 5.9 | 8.7 | 700 |
| 9 | - | 2-18 | 410 | 6.0 | 8.6 | 630 |
| 10 | - | 2-29 | 410 | 7.6 | 6.8 | 560 |

A comparison between Table 1 and Table 2 shows that Examples 1 to 21 had improved luminance and lifetime characteristics, and exhibited excellent characteristics.

### Example 22

A thin film was laminated by a vacuum deposition method at a degree of vacuum of 4.0× 10⁻⁴ Pa on a glass substrate having formed thereon an anode comprising indium tin oxide (ITO) having a thickness of 150 nm. First, copper phthalocyanine (CuPC) was formed into a layer having a thickness of 20 nm to serve as a hole-injecting layer on the ITO. Next, NPB was formed into a layer having a thickness of 20 nm to serve as a hole-transporting layer. Next, compound 1-2 serving as a first host, compound 2-1 serving as a second host for a light-emitting layer, and tris(2-phenylpyridine)iridium (III) (Ir(PPy)₃) serving as a light-emitting layer guest were co-deposited from different deposition sources to form a light-emitting layer having a thickness of 30 nm. At this time, a vapor deposition rate ratio among the first host, the second host, and Ir(PPy)₃ was 47:47:6. Next, aluminum (III) bis(2-methyl-8-quninolinato)-4-phenylphenolate (BAlq) was formed into a layer having a thickness of 10 nm to serve as a hole-blocking layer. Next, Alq3 was formed into a layer having a thickness of 40 nm to serve as an electron-transporting layer. Further, lithium fluoride (LiF) was formed into a layer having a thickness of 0.5 nm to serve as an electron-injecting layer on the electron-transporting layer. Finally, Al was formed into a layer having a thickness of 100 nm to serve as a cathode on the electron-injecting layer to produce an organic EL device.

An external power source was connected to the resultant organic EL device and a DC voltage was applied to the device. As a result, an emission spectrum having a local maximum wavelength of 517 nm was observed and it was found that light emission from Ir(PPy)₃ was obtained. Table 2 shows the properties (luminance, voltage, luminance efficiency, and luminance half-time) of the produced organic EL device.

### Examples 23 to 42

Organic EL devices were each produced in the same manner as in Example 22 except that in Example 22, a compound shown in Table 2 was used as the first host of the light-emitting layer (Examples 23 to 28).

Organic EL devices were each produced in the same manner as in Examples 22 to 28 except that compound 2-18 or 2-29 was used as the second host of the light-emitting layer (Examples 29 to 42).

An external power source was connected to the resultant organic EL devices, and a DC voltage was applied to the devices. As a result, an emission spectrum having a local maximum wavelength of 517 nm was observed and it was found that light emission from Ir(PPy)₃ was obtained. Table 3 shows the properties of the produced organic EL devices.

### Comparative Examples 11 to 20

Organic EL devices were each produced in the same manner as in Example 22 except that in Example 22, a compound shown in Table 2 was used alone as the light-emitting layer host. The host amount was set to the same amount as the total of the first host and second host in Example 22, and the guest amount was the same. A power source was connected to each of the resultant organic EL devices and a DC voltage was applied to the device. As a result, an emission spectrum having a local maximum wavelength of 517 nm was observed for each of the organic EL devices and it was found that light emission from Ir(PPy)₃ was obtained. Table 4 shows the properties of the produced organic EL devices.

In Tables 3 and 4, the luminance, the voltage, and the luminous efficacy are values at a driving current of 20 mA/cm², and the luminance half-time is a value at an initial luminance of 1,000 cd/m².

**[Table 3]**

| Example | 1st host compound No. | 2nd host compound No. | Luminance (cd/m²) | Voltage (V) | Luminous efficiency (lm/W) | Luminance half-time (h) |
|---|---|---|---|---|---|---|
| 22 | 1-2 | 2-1 | 8900 | 4.2 | 33.1 | 12000 |
| 23 | 1-8 | | 8900 | 4.2 | 33.5 | 12000 |
| 24 | 1-11 | | 9000 | 4.2 | 33.3 | 12000 |
| 25 | 1-15 | | 8900 | 4.2 | 33.4 | 20000 |
| 26 | 1-44 | | 8900 | 4.2 | 33.2 | 14000 |
| 27 | 1-45 | | 8900 | 4.3 | 32.8 | 18000 |
| 28 | 1-68 | | 9000 | 4.3 | 33.2 | 14000 |
| 29 | 1-2 | 2-18 | 8700 | 4.2 | 32.2 | 10800 |
| 30 | 1-8 | | 8700 | 4.2 | 32.6 | 10800 |
| 31 | 1-11 | | 8800 | 4.3 | 32.4 | 10800 |
| 32 | 1-15 | | 8700 | 4.2 | 32.5 | 18000 |
| 33 | 1-44 | | 8700 | 4.2 | 32.3 | 12600 |
| 34 | 1-45 | | 8700 | 4.3 | 32.0 | 16200 |
| 35 | 1-68 | | 8800 | 4.3 | 32.4 | 12600 |
| 36 | 1-2 | 2-29 | 9000 | 4.2 | 33.5 | 9600 |
| 37 | 1-8 | | 9000 | 4.2 | 33.9 | 9600 |
| 38 | 1-11 | | 9100 | 4.2 | 33.7 | 9600 |
| 39 | 1-15 | | 9000 | 4.2 | 33.8 | 16000 |
| 40 | 1-44 | | 9000 | 4.2 | 33.5 | 11200 |
| 41 | 1-45 | | 9100 | 4.3 | 33.6 | 14400 |
| 42 | 1-68 | | 9100 | 4.3 | 33.6 | 11200 |

**[Table 4]**

| Comp. Ex. | 1st host compound No. | 2nd host compound No. | Luminance (cd/m²) | Voltage (V) | Luminous efficiency (lm/W) | Luminance half-time (h) |
|---|---|---|---|---|---|---|
| 11 | 1-2 | - | 7600 | 4.7 | 25.6 | 1810 |
| 12 | 1-8 | - | 7700 | 5.2 | 23.0 | 1810 |
| 13 | 1-11 | - | 7500 | 4.5 | 26.2 | 1810 |
| 14 | 1-15 | - | 7600 | 5.1 | 23.6 | 2700 |
| 15 | 1-44 | - | 7600 | 4.8 | 25.2 | 2100 |
| 16 | 1-45 | - | 7600 | 4.4 | 27.2 | 2700 |
| 17 | 1-68 | - | 7500 | 4.4 | 26.7 | 2100 |
| 18 | - | 2-1 | 7600 | 4.5 | 26.8 | 3000 |
| 19 | - | 2-18 | 7500 | 4.5 | 26.4 | 2700 |
| 20 | - | 2-29 | 7700 | 5.1 | 23.7 | 2400 |

A comparison between Table 3 and Table 4 shows that Examples 22 to 42 had improved luminance and life characteristics, and exhibited excellent characteristics.

### Example 43

A thin film was laminated by a vacuum deposition method at a degree of vacuum of 2.0 × 10⁻⁵ Pa on a glass substrate having formed thereon an anode comprising indium tin oxide (ITO) having a thickness of 70 nm. First, copper phthalocyanine (CuPC) was formed into a layer having a thickness of 30 nm to serve as a hole-injecting layer on the ITO. Next, NPD was formed into a layer having a thickness of 15 nm to serve as a hole-transporting layer. Next, mCBP serving as a host material for the light-emitting layer and FIrpic serving as a dopant were co-deposited from different deposition sources onto the hole-transporting layer to form a light-emitting layer having a thickness of 30 nm. The concentration of FIrpic was 20 wt%. Next, compound 1-15 (compound A) and compound 2-1(compound B) were co-deposited from different deposition sources onto the light-emitting layer to form a hole-blocking layer having a thickness of 5 nm. At this time, a vapor deposition rate ratio of compound 1-15 to compound 2-1 was 50:50 (by weight). Next, Alq3 was formed into a layer having a thickness of 20 nm to serve as an electron-transporting layer. Further, LiF was formed into a layer having a thickness of 1.0 nm to serve as an electron-injecting layer on the electron-transporting layer. Finally, Al was formed into a layer having a thickness of 70 nm to serve as an electrode on the electron-injecting layer.

The resulting organic EL device has a layer construction comprising the electron-injecting layer added between the cathode and the electron-transporting layer, and the hole-blocking layer added between the light-emitting layer and the electron-transporting layer in the organic EL device illustrated in FIG. 1. An external power source was connected to the resultant organic EL device and a DC voltage was applied to the device. As a result, an emission spectrum having a local maximum wavelength of 475 nm was observed and it was found that light emission from FIrpic was obtained. Table 5 shows the properties of the produced organic EL device.

### Examples 44 to 48

Organic EL devices were each produced in the same manner as in Example 43 except that compound 2-18 or 2-29 was used in place of compound 2-1 in Example 43 as compound B for the hole-blocking layer (Examples 44 and 45).

Organic EL devices were each produced in the same manner as in Example 43 to 45 except that compound 1-15 was used in place of compound 1-45 as compound A for the hole-blocking layer (Examples 46 to 48).

An external power source was connected to each of the resultant organic EL devices and a DC voltage was applied to the device. As a result, an emission spectrum having a local maximum wavelength of 475 nm was observed for each of the organic EL devices and it was found that light emission from FIrpic was obtained. Table 5 shows the properties of each of the produced organic EL devices.

### Comparative Example 21

An organic EL device was produced in the same manner as in Example 43 in the film thickness of Alq₃ serving as the electron-transporting layer in Example 43 was 25 nm and no hole-blocking layer was provided.

In Table 5, the luminance, the voltage, and the luminous efficacy are values at a driving current of 2.5 mA/cm², and the luminance half-time is a value at an initial luminance of 1,000 cd/m².

**[Table 5]**

| Example | Compound A No. | Compound B No. | Luminance (cd/m²) | Voltage (V) | Luminous efficiency (lm/W) | Luminance half-time (h) |
|---|---|---|---|---|---|---|
| 43 | 1-15 | 2-1 | 620 | 6.1 | 12.7 | 1500 |
| 44 | | 2-18 | 600 | 6.9 | 10.8 | 1350 |
| 45 | | 2-29 | 590 | 5.0 | 14.9 | 1200 |
| 46 | 1-45 | 2-1 | 620 | 7.0 | 11.0 | 1500 |
| 47 | | 2-18 | 580 | 7.2 | 10.0 | 1500 |
| 48 | | 2-29 | 590 | 6.2 | 11.9 | 1050 |
| Comp.Ex. 21 | - | - | 520 | 9.4 | 7.0 | 300 |

Table 5 shows that Example 43 to 48 comprising two compounds used for the hole-blocking layer exhibited excellent characteristics, as compared to Comparative Example 21 comprising no hole-blocking material.

### INDUSTRIAL APPLICABILITY

The organic EL device of the present invention has high luminous efficacy at a low driving voltage and a long life, and is expected to be applied to full-color or multi-color panels. The organic EL device of the present invention can be used for mobile device displays, and also can be used for organic EL displays or organic EL lighting devices of TV sets or automobiles.

### REFERENCE SIGNS LIST

1 substrate
2 anode
3 hole-injecting layer
4 hole-transporting layer
5 light-emitting layer
6 electron-transporting layer
7 electron-injecting layer
8 cathode

## Claims

1. An organic electroluminescent device comprising a substrate having stacked thereon an anode, organic layers, and a cathode, wherein at least one layer of the organic layers comprises (i) a compound represented by the following general formula (1) and (ii) a compound represented by the following general formula (2): 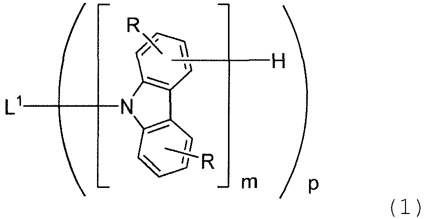
wherein L¹ is a substituted or unsubstituted *p*-valent aromatic hydrocarbon group having 6 to 30 carbon atoms, substituted or unsubstituted a *p*-valent heteroaromatic ring group having 3 to 30 carbon atoms other than a carbazolyl group, or a *p*-valent linked aromatic group obtained by linking 2 to 6 aromatic rings of the substituted or unsubstituted aromatic rings,
each R independently represents hydrogen, a substituted or unsubstituted aromatic hydrocarbon group having 6 to 30 carbon atoms, a substituted or unsubstituted heteroaromatic ring group having 3 to 30 carbon atoms other than a carbazolyl group, a linked aromatic group obtained by linking 2 to 6 aromatic rings of the substituted or unsubstituted aromatic hydrocarbon groups or heteroaromatic ring groups, a linear, branched, or cyclic alkyl group having 1 to 12 carbon atoms, a diarylamino group having 12 to 44 carbon atoms, a cyano group, a nitro group, or a fluoro group,
*p* is a substitution number and represents an integer of 1 to 3, *m* is a repeating number, and each *m* is independently an integer of 2 to 4, 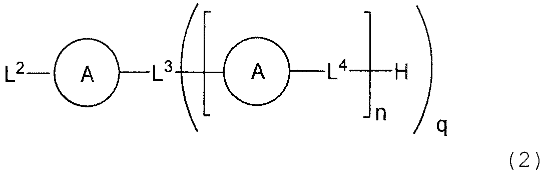 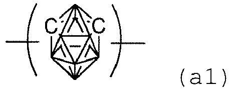 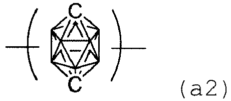
wherein ring A is a divalent carborane group of C₂B₁₀H₁₀ represented by formula (a1) or formula (b1), with the proviso that when a plurality of rings A are present in a molecule, the plurality of rings A may be the same or different from each other;
*q* is a substitution number and is an integer of 1 to 4;
*n* is a repeating number and is an integer of 0 to 2;
L² represents a substituted or unsubstituted aromatic hydrocarbon group having 6 to 30 carbon atoms, a substituted or unsubstituted heteroaromatic ring group having 3 to 30 carbon atoms, or a linked aromatic group obtained by linking 2 to 6 aromatic rings of the substituted or unsubstituted aromatic hydrocarbon groups or
heteroaromatic ring groups,
L³ represents a single bond, a substituted or unsubstituted (*q* + 1)-valent aromatic hydrocarbon group having 6 to 30 carbon atoms,
a substituted or unsubstituted (*q* + 1)-valent heteroaromatic group having 3 to 30 carbon atoms, or a (*q*+ 1)-valent linked aromatic group obtained by linking 2 to 6 aromatic rings of the substituted or
unsubstituted aromatic hydrocarbon groups or heteroaromatic ring groups, with the proviso that when *q* = 1 and *n* = 1, L³ represents a single bond, a heteroaromatic ring group, or a linked aromatic group comprising at least one heteroaromatic ring group, and
L⁴ independently represents a single bond, a substituted or
unsubstituted divalent aromatic hydrocarbon group having 6 to 30 carbon atoms, a substituted or unsubstituted divalent heteroaromatic ring group having 3 to 30 carbon atoms, or a substituted or
unsubstituted linked aromatic group obtained by linking 2 to 6 aromatic groups of the aromatic hydrocarbon groups or the heteroaromatic ring groups.

2. The organic electroluminescent device according to claim 1, wherein, in general formula (1), *p* is an integer of 1 or 2, *m* is independently an integer of 2 or 3, and all binding structures between carbazolyl groups are represented by formula (d1) or both of formula (c1) and formula (d1). 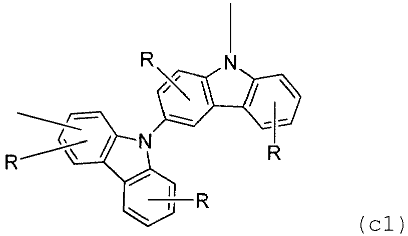 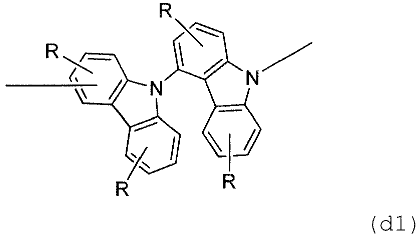 wherein R is the same as in general formula (1).

3. The organic electroluminescent device according to claim 2, wherein, in general formula (1), all binding structures between carbazolyl groups are represented by both of formula (c1) and formula (d1).

4. The organic electroluminescent device according to claim 1, wherein, in general formula (1), L¹ is a *p*-valent group formed by removing *p* hydrogen atoms from any one of formulae (3) to (6). 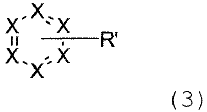 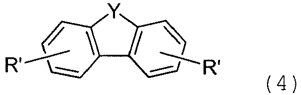 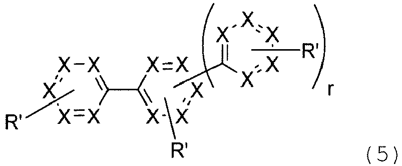 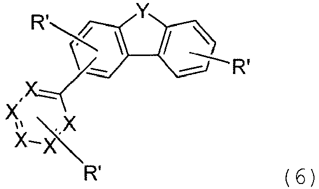
in formulae (3) to (6), each X independently represents CH or nitrogen, each R' independently represents hydrogen, a substituted or unsubstituted aromatic hydrocarbon group having 6 to 30 carbon atoms, a substituted or unsubstituted heteroaromatic ring group having 3 to 30 carbon atoms, an alkyl group having 1 to 12 carbon atoms, a diarylamino group having 12 to 44 carbon atoms, a cyano group, a nitro group, or a fluoro group;
in formulae (4) and (6), Y represents oxygen or sulfur, and in formulae (5), r represents an integer of 0 to 2.

5. The organic electroluminescent device according to claim 4, wherein, in general formula (1), L¹ is a *p*-valent group formed by removing p hydrogen atoms from any one of formulae (3), (4), and (5).

6. The organic electroluminescent device according to claim 1, wherein, in general formula (1), the total of *m* is an integer of 2 to 6.

7. The organic electroluminescent device according to claim 1, wherein, in general formula (2), ring A is a divalent carborane group of C₂B₁₀H₈ represented by formula (a1).

8. The organic electroluminescent device according to claim 1, wherein, in general formula (2), aromatic rings of L² and L³, which directly bond to ring A, are the same.

9. The organic electroluminescent device according to claim 1, wherein, in general formula (2), L² and L³ are a substituted or unsubstituted dibenzofuranyl group or a substituted or unsubstituted dibenzothiophenyl group.

10. The organic electroluminescent device according to any of claims 1 to 9, wherein an organic layer comprising a compound represented by general formula (1) and a compound represented by general formula (2) is at least one layer selected from the group consisting of a light-emitting layer containing a luminescent dopant, an electron-blocking layer, and a hole-blocking layer.

11. The organic electroluminescent device according to claim 10, wherein the organic layer is a light-emitting layer containing a luminescent dopant and comprises a compound represented by general formula (1) and a compound represented by general formula (2) as host materials.

12. The organic electroluminescent device according to claim 11, wherein the luminescent dopant is a delayed fluorescent dopant.

13. The organic electroluminescent device according to claim 11, the luminescent dopant is an organometallic complex comprising at least one metal selected from ruthenium, rhodium, palladium, silver, rhenium, osmium, iridium, platinum, and gold.
